# EUROPEAN PATENT APPLICATION

(11) **EP 4 830 627 A1**
(43) Date of publication of application: **28.10.2026**
(21) Application number: 24910334.2
(22) Date of filing: 08.11.2024
(51) Int. Cl.: A61B 5/18

(54) **USER WORKLOAD STATE PROCESSING METHOD AND APPARATUS BASED ON MULTIMODAL DATA, AND DEVICE**

(30) Priority: 28.12.2023 CN 202311841098; 29.12.2023 CN 202311865043
(71) Applicant: Kingfar International Inc., Beijing 100085 (CN)
(72) Inventor: ZHAO, Qichao, Beijing 100085 (CN); YANG, Ran, Beijing 100085 (CN); WANG, Qingju, Beijing 100085 (CN)
(74) Representative: Meyer-Dulheuer MD Legal Patentanwälte PartG mbB
(86) International application number: PCT/CN2024/130736
(87) International publication number: WO 2025/139397

(57) **Abstract**

Provided are a method, and an apparatus for processing a personnel workload state based on multimodal data, and a device. The method includes: acquiring state information of a first user; determining workload information of the first user according to the acquired state information, the state information includes at least two of: physiological information, eye movement information, electroencephalogram information, brain function imaging information, motion capture information, spatiotemporal acquisition information, behavior acquisition information, and facial expression and state information; and feeding back the workload information to a first management account, and formulating a training scheme matching the workload information. According to the embodiments of the present disclosure, the workload of the user can be recognized based on the multimodal data of the user.

## Description

The present application claims priority to Chinese Patent Application No. 202311841098.9, entitled "Method and Apparatus for Processing Personnel Workload State Based on Multimodal Data, and Device", filed on December 28, 2023, the content of which is incorporated herein by reference in its entirety.

The present application claims priority to Chinese Patent Application No. 202311865043.1, entitled "Method and Apparatus for Recognizing Pilot Workload based on Human-Factor Intelligence, and Device", filed on December 29, 2023, the content of which is incorporated herein by reference in its entirety.

### TECHNICAL FIELD

The present disclosure relates to the field of physiological signal recognition technologies, and in particular, to a method and an apparatus for processing a workload state based on multimodal data, and a device.

### BACKGROUND

Workload refers to the amount of work imposed on a human body per unit time. When the workload is relatively high, the working capacity of the worker is at a high level. At this time, both the physiological and psychological aspects of the worker will incur substantial consumption. As the remaining working capacity is limited, the worker is prone to various accidents due to the inability to cope with unexpected events. Therefore, timely recognizing the workload of the worker during the working process and making corresponding adjustments is an effective way to ensure work safety.

### SUMMARY

The present disclosure provides a method and an apparatus for processing a workload state based on multimodal data, and a device, which can recognize the workload of a user based on the multimodal data of the user.

In a first aspect, an embodiment of the present disclosure provides a method for processing a workload state based on multimodal data, including: acquiring state information of a first user; determining workload information of the first user based on the acquired state information, the state information includes at least two of: physiological information, eye movement information, electroencephalogram information, brain function imaging information, motion capture information, spatiotemporal acquisition information, behavior acquisition information, and facial expression and state information; and feeding back the workload information to a first management account, and formulating a training scheme matching the workload information.

According to the method, the state information of the first user is acquired, and the workload information of the first user is determined according to the state information, thereby realizing recognition of the workload of the user based on the multimodal state information of the first user. In addition, the workload information is fed back to the first management account, and a training scheme matching the workload information is formulated, so that the training scheme can be adapted to the workload information of the first user, thereby ensuring the scientificity and safety of the training of the first user.

In a possible implementation, when the state information of the first user includes a first electroencephalogram signal and a first near-infrared brain function imaging signal of the first user, the determining workload information of the first user based on the acquired state information includes: respectively extracting a first feature of the first electroencephalogram signal and a second feature of the first near-infrared brain function imaging signal; and determining the workload information of the first user based on the first feature and the second feature.

In a possible implementation, the determining the workload information of the first user based on the first feature and the second feature includes: fusing the first feature and the second feature to obtain a first fusion feature; and inputting the first fusion feature into a preset workload recognition model, and taking workload information output by the workload recognition model as the workload information of the first user.

In a possible implementation, the first feature includes a first temporal feature and a first image feature, and the second feature includes a second temporal feature and a second image feature. The fusing the first feature and the second feature to obtain a first fusion feature includes: fusing the first temporal feature and the second temporal feature to obtain a third temporal feature; fusing the first image feature and the second image feature to obtain a third image feature; and fusing the third temporal feature and the third image feature to obtain the first fusion feature.

In a possible implementation, the fusing the first and the second temporal feature to obtain a third temporal feature includes: converting the first temporal feature into a fourth image feature; converting the second temporal feature into a fifth image feature; and fusing the fourth image feature and the fifth image feature to obtain a sixth image feature, and taking the sixth image feature as the third temporal feature.

In a possible implementation, the first temporal feature includes: a time-domain feature, a frequency-domain feature, a time-frequency-domain feature, a nonlinear feature, and/or a brain function connection feature. Additionally/alternatively, the first image feature includes: a two-dimensional (2D) map and/or a three-dimensional (3D) dense connection network. Additionally/alternatively, the second temporal feature includes: concentrations of oxygenated and deoxygenated hemoglobin, a β-index of a single channel, and/or a Pearson correlation coefficient between channels. Additionally/alternatively, the second image feature includes: a 3D channel activation map, a correlation matrix formed between channels, and/or a brain network connection map.

In a possible implementation, the workload information includes: a workload level and/or a workload type.

In a possible implementation, the first feature includes a first temporal feature and a first image feature, the second feature includes a second temporal feature and a second image feature, and the workload information includes a workload level and a workload type. The determining the workload information of the first user based on the first feature and the second feature includes: fusing the first temporal feature and the second temporal feature to obtain a third temporal feature, and recognizing the workload level of the first user based on the third temporal feature; and fusing the first image feature and the second image feature to obtain a third image feature, and recognizing the workload type of the first user based on the third image feature.

In a possible implementation, the recognizing the workload level of the first user based on the third includes: inputting the third temporal feature into a load level recognition model, and taking a workload level output by the load level recognition model as the workload level of the first user.

In a possible implementation, the recognizing the workload type of the first user based on the third image feature includes: inputting the third image feature into a load type recognition model, and taking a workload type output by the load type recognition model as the workload type of the first user.

In a possible implementation, the first temporal feature includes: a time-domain feature, a frequency-domain feature, a time-frequency-domain feature, a nonlinear feature, and/or a brain function connection feature. Additionally/alternatively, the first image feature includes a brain topographic map and/or a power spectrum topographic map. Additionally/alternatively, the second temporal feature includes: concentrations of oxygenated and deoxygenated hemoglobin, a β-index of a single channel, and/or a Pearson correlation coefficient between channels. Additionally/alternatively, the second image feature includes: a 3D channel activation map, a complex brain network, a default brain network, a correlation heat map, and/or a brain network connection map.

In a possible implementation, the workload type includes: auditory, visual, attention, executive, and/or planning.

In a possible implementation, extracting the second image feature of the first near-infrared brain function imaging signal includes: extracting brain network maps with different functions from the first near-infrared brain function imaging signal as the second image feature.

In a possible implementation, before respectively extracting a first feature of the first electroencephalogram signal and a second feature of the first near-infrared brain function imaging signal, the method further includes: respectively preprocessing on the first electroencephalogram signal and the first near-infrared brain function imaging signal.

In a possible implementation, the method further includes: performing a warning processing on user workload based on the workload information.

In a possible implementation, the performing a warning processing on user workload based on the workload information includes: when the workload level in the workload information is not lower than a preset level, and the workload type in the workload information includes a first type, alarming for the workload type of the first type.

In a possible implementation, the performing a warning processing on user workload based on the workload information includes: when the workload level in the workload information is not lower than a preset level and the workload type in the workload information includes a second type, determining a proportion of the second type among a plurality of workload information obtained within a preset first duration, and when the proportion is not lower than a preset proportion threshold, alarming for the workload type of the second type.

In a possible implementation, the formulating a training scheme matching the workload information includes: obtaining a training scheme corresponding to the workload information as the training scheme of the first user.

In a possible implementation, the method for training a workload recognition model includes: acquiring a second electroencephalogram signal, a second near-infrared brain function imaging signal, and a workload information label of a second user, where the workload information label is configured to record workload information of the second user when acquiring the second electroencephalogram signal and the second near-infrared brain function imaging signal of the second user; respectively extracting a first feature of the second electroencephalogram signal and a second feature of the second near-infrared brain function imaging signal; fusing the first feature of the second electroencephalogram signal and the second feature of the second near-infrared brain function imaging signal to obtain a second fusion feature; and training a workload recognition model using the second fusion feature and the workload information label as samples.

In a possible implementation, the workload recognition model includes a plurality of network layers. The training a workload recognition model using the second fusion feature and the workload information label as samples includes: inputting the second fusion feature into the workload recognition model to obtain an output result of each network layer during a forward propagation process; determining a target result of each network layer based on the workload information label; and for each network layer, for each network layer, calculating a similarity between the output result and the target result of the network layer, and adjusting a weight of the network layer based on the similarity.

In a possible implementation, a method for training a load level recognition model includes: acquiring a third electroencephalogram signal, a third near-infrared brain function imaging signal, and a workload level label of a third user, where the workload level label is configured to record a workload level of the third user when acquiring the third electroencephalogram signal and the third near-infrared brain function imaging signal of the third user; respectively extracting a fourth temporal feature of the third electroencephalogram signal, and extracting a fifth temporal feature of the third near-infrared brain function imaging signal; fusing the fourth temporal feature of the third electroencephalogram signal and the fifth temporal feature of the third near-infrared brain function imaging signal to obtain a sixth temporal feature; and training the load level recognition model using the sixth temporal feature and the workload level label as samples.

In a possible implementation, a method for training a load type recognition model includes: acquiring a third electroencephalogram signal, a third near-infrared brain function imaging signal, and a workload type label of a third user, where the workload type label is configured to record a workload type of the third user when acquiring the third electroencephalogram signal and the third near-infrared brain function imaging signal of the third user; respectively extracting a fourth image feature of the third electroencephalogram signal, and extracting a fifth image feature of the third near-infrared brain function imaging signal; fusing the fourth image feature of the third electroencephalogram signal and the fifth image feature of the third near-infrared brain function imaging signal to obtain a sixth image feature; and training the load type recognition model using the sixth image feature and the workload type label as samples.

In a second aspect, an embodiment of the present disclosure provides an apparatus for processing a workload state based on multimodal data, including: an acquisition module configured to acquire state information of a first user; a determination module configured to determine workload information of the first user based on the acquired state information, where the state information includes at least two of: physiological information, eye movement information, electroencephalogram information, brain function imaging information, motion capture information, spatiotemporal acquisition information, behavior acquisition information, and facial expression and state information; and a processing module configured to feed back the workload information to a first management account, and formulate a training scheme matching the workload information.

In a possible implementation, when the state information of the first user includes a first electroencephalogram signal and a first near-infrared brain function imaging signal of the first user; the determination module may include: a feature extraction module configured to respectively extract a first feature of the first electroencephalogram signal and a second feature of the first near-infrared brain function imaging signal; and an information determination module configured to determine the workload information of the first user based on the first feature and the second feature.

In a possible implementation, the information determination module includes: a feature fusion module configured to fuse the first feature and the second feature to obtain a first fusion feature; and a recognition module configured to input the first fusion feature into a preset workload recognition model, and take workload information output by the workload recognition model as the workload information of the first user.

In a possible implementation, the acquisition module is further configured to: acquire a second electroencephalogram signal, a second near-infrared brain function imaging signal, and a workload information label of a second user, where the workload information label is configured to record workload information of the second user when acquiring the second electroencephalogram signal and the second near-infrared brain function imaging signal of the second user. The feature extraction module is further configured to: respectively extract a first feature of the second electroencephalogram signal and a second feature of the second near-infrared brain function imaging signal. The feature fusion module is further configured to: fuse the first feature of the second electroencephalogram signal and the second feature of the second near-infrared brain function imaging signal to obtain a second fusion feature. The apparatus further includes: a first training module configured to train a workload recognition model using the second fusion feature and the workload information label as samples.

In a possible implementation, the information determination module includes: a first fusion module configured to fuse the first temporal feature and the second temporal feature to obtain a third temporal feature; a first recognition module configured to recognize a workload level of the first user based on the third temporal feature; a second fusion module configured to fuse the first image feature and the second image feature to obtain a third image feature; and a second recognition module configured to recognize the workload type of the first user based on the third image feature.

In a possible implementation, the acquisition module is further configured to: acquire a third electroencephalogram signal, a third near-infrared brain function imaging signal, and a workload level label of a third user, where the workload level label is configured to record a workload level of the third user when acquiring the third electroencephalogram signal and the third near-infrared brain function imaging signal of the third user. The feature extraction module is further configured to: respectively extract a fourth temporal feature of the third electroencephalogram signal, and extract a fifth temporal feature of the third near-infrared brain function imaging signal. The first fusion module is further configured to fuse the fourth temporal feature of the third electroencephalogram signal and the fifth temporal feature of the third near-infrared brain function imaging signal to obtain a sixth temporal feature. The apparatus further includes: a second training module configured to train the load level recognition model using the sixth temporal feature and the workload level label as samples.

In a possible implementation, the acquisition module is further configured to acquire a third electroencephalogram signal, a third near-infrared brain function imaging signal, and a workload type label of a third user, where the workload type label is configured to record a workload type of the third user when acquiring the third electroencephalogram signal and the third near-infrared brain function imaging signal of the third user. The feature extraction module is further configured to respectively extract a fourth image feature of the third electroencephalogram signal, and extract a fifth image feature of the third near-infrared brain function imaging signal. The second fusion module is further configured to fuse the fourth image feature of the third electroencephalogram signal and the fifth image feature of the third near-infrared brain function imaging signal to obtain a sixth image feature. The apparatus further includes: a third training module configured to: train the load type recognition model using the sixth image feature and the workload type label as samples.

In a third aspect, an embodiment of the present disclosure provides an electronic device, including: a processor, and a memory, one or more computer programs are stored in the memory, the one or more computer programs include instructions, and the electronic device, when executed by the processor, is configured to implement the method according to any one of the first aspect.

In a fourth aspect, an embodiment of the present disclosure provides a computer-readable storage medium on which a computer program is stored. The computer program, when running on a computer, enable the computer to implement the method according to the first aspect.

### BRIEF DESCRIPTION OF DRAWINGS

In order to more clearly describe the technical solutions of embodiments of the present disclosure, the following briefly describes the drawings desired in the embodiments. It is appreciated that, the drawings described below are merely some embodiments of the present disclosure, and for those skilled in the art, other drawings can be obtained based on these drawings without creative effort.
FIG. 1A is a schematic structural diagram of a system architecture applicable to a method for processing a workload state based on multimodal data according to an embodiment of the present disclosure;
FIG. 1B is a schematic structural diagram of an electronic device according to an embodiment of the present disclosure;
FIG. 2 is a schematic flowchart of a method for processing a workload state based on multimodal data according to an embodiment of the present disclosure;
FIG. 3 is another schematic flowchart of a method for processing a workload state based on multimodal data according to an embodiment of the present disclosure;
FIG. 4A is another schematic flowchart of a method for processing a workload state based on multimodal data according to an embodiment of the present disclosure;
FIG. 4B is another schematic flowchart of a method for processing a workload state based on multimodal data according to an embodiment of the present disclosure;
FIG. 5 is a schematic flowchart of a method for training a workload recognition model according to an embodiment of the present disclosure;
FIG. 6 is another schematic flowchart of a method for processing a workload state based on multimodal data according to an embodiment of the present disclosure;
FIG. 7 is a schematic flowchart of a method for recognizing pilot workload based on human-factor intelligence according to an embodiment of the present disclosure;
FIG. 8 is another schematic flowchart of a method for recognizing pilot workload based on human-factor intelligence according to an embodiment of the present disclosure;
FIG. 9 is another schematic flowchart of a method for recognizing pilot workload based on human-factor intelligence according to an embodiment of the present disclosure;
FIG. 10 is another schematic flowchart of a method for recognizing pilot workload based on human-factor intelligence according to an embodiment of the present disclosure;
FIG. 11 is a schematic flowchart of a method for training a load type recognition model according to an embodiment of the present disclosure;
FIG. 12 is another schematic flowchart of a method for training a load level recognition model according to an embodiment of the present disclosure;
FIG. 13 is another schematic flowchart of a method for recognizing pilot workload based on human-factor intelligence according to an embodiment of the present disclosure;
FIG. 14 is a schematic structural diagram of an apparatus for processing a workload state based on multimodal data according to an embodiment of the present disclosure;
FIG. 15 is another schematic structural diagram of an apparatus for processing a workload state based on multimodal data according to an embodiment of the present disclosure;
FIG. 16 is another schematic structural diagram of an apparatus for processing a workload state based on multimodal data according to an embodiment of the present disclosure;
FIG. 17 is a schematic structural diagram of an apparatus for recognizing workload according to an embodiment of the present disclosure; and
FIG. 18 is another schematic structural diagram of an apparatus for recognizing workload according to an embodiment of the present disclosure.

### DESCRIPTION OF EMBODIMENTS

Part of terms used in the embodiments of the present disclosure is merely used to explain specific embodiments of the present disclosure, and are not intended to limit the present disclosure.

Workload refers to the amount of work imposed on a human body per unit time. When the workload is relatively high, the working capacity of the worker is at a high level. At this time, both the physiological and psychological aspects will incur substantial consumption. As the remaining working capacity is limited, the worker is prone to various accidents due to the inability to cope with unexpected events. Therefore, timely recognizing the workload of the worker during the working process and making corresponding adjustments is an effective way to ensure work safety. In particular, in scenarios where high work safety requirements are imposed on workers, such as a driver driving a vehicle, a pilot flying an airplane, and a pilot conducting flight training, it is even more necessary to timely recognize the workload of workers during work, so as to ensure work safety.

Electroencephalogram data acquires electrical activity of the cerebral cortex, and features a millisecond-level temporal resolution, enabling extraction of diverse and informative features with high sensitivity. In addition, with the development of sensor technologies, types of portable electroencephalogram devices are gradually diversified, which are widely used in fields such as biofeedback, emotion detection, state recognition, and brain-computer interfaces.

Accordingly, embodiments of the present disclosure provide a method for processing a workload state based on multimodal data, which recognize the workload of the user by utilizing the multimodal data of the user, such as the electroencephalogram data, thereby improving the work safety of the user.

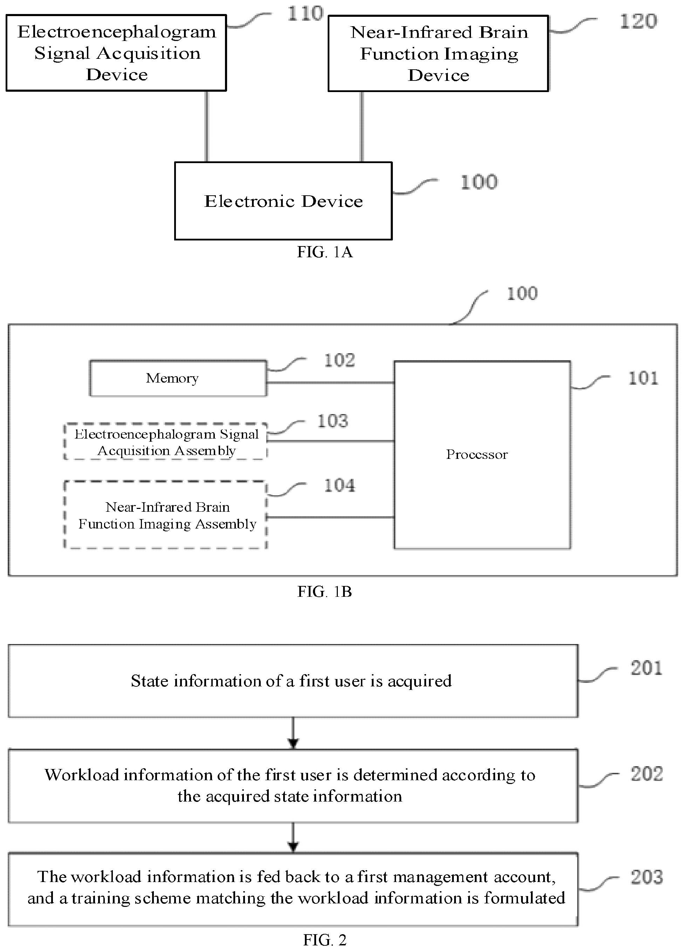

FIG. 1A is a schematic structural diagram of a system architecture applicable to a method for processing a workload state based on multimodal data according to an embodiment of the present disclosure. As shown in FIG. 1A, the system may include: an electronic device 100, an electroencephalogram signal acquisition device 110, and a near-infrared brain function imaging device 120.

The electroencephalogram signal acquisition device 110 is configured to acquire an electroencephalogram signal of a user, and transmit the electroencephalogram signal of the user to the electronic device 100.

The near-infrared brain function imaging device 120 is configured to acquire a near-infrared brain function imaging signal of a user, and transmit the near-infrared brain function imaging signal of the user to the electronic device 100.

In some embodiments, the electroencephalogram signal acquisition device 110 and the near-infrared brain function imaging device 120 may also be implemented by a multimodal brain function imaging device having both functions of electroencephalogram signal acquisition and near-infrared brain function imaging signal acquisition.

The electronic device 100 is configured to perform the method for processing the workload state based on multimodal data according to the embodiments of the present disclosure, to implement workload recognition of the user.

In some embodiments, the method for processing the workload state based on multimodal data in the embodiments of the present disclosure may be applicable to recognizing the workload of a working user such as a driver or a pilot. Taking the recognition of the workload of a driver as an example, the system shown in FIG. 1A may be a driver-assistance system. Taking the recognition of the workload of a pilot as an example, the system shown in FIG. 1A may be a flight-assistance system or a flight simulation system.

As shown in FIG. 1B, which is a schematic structural diagram of an electronic device 100 according to an embodiment of the present disclosure, the electronic device 100 includes: a processor 110, a memory 120, etc.

In an example, to provide more complete functionality, the electronic device may further include one or more components such as a display screen, a camera, a loudspeaker, an antenna, a mobile communication module, a wireless communication module, an audio module, an earpiece receiver, a microphone, an earphone interface, a charging management module, a power management module, and a battery, which is not limited in the embodiments of the present disclosure.

The processor 110 may include one or more processing units. For example: the processor 110 may include an application processor (AP), a modem processor, a graphics processing unit (GPU), an image signal processor (ISP), a controller, a video codec, a digital signal processor (DSP), a baseband processor, a neural-network processing unit (NPU), and/or the like. Different processing units may be independent devices, or may be integrated into one or more processors.

A memory may also be provided within the processor 110 for storing instructions and data. In some embodiments, the memory within the processor 110 is a high-speed cache memory. The memory may store instructions or data recently used or used repeatedly by the processor 110. If the processor 110 needs to use the instructions or data again, it may be directly called from the memory, thereby avoiding redundant access, reducing the waiting time of the processor 110, and improving the efficiency of the system.

The memory 120 may be configured to store computer-executable program codes including instructions. The memory 120 may include a program storage area and a data storage area. The program storage area may store an operating system, and an application program required by at least one function (such as a sound playback function and an image playback function), etc. The data storage area may store data (such as audio data) created during the use of the electronic device 100, etc. In addition, the memory 120 may include a high-speed random access memory, and may further include a non-volatile memory, such as at least one magnetic disk storage device, a flash memory device, or a universal flash storage (UFS). The processor 110 executes various functional applications and data processing of the electronic device 100 by running instructions stored in the memory 120 and/or instructions stored in the memory provided in the processor.

It should be noted that, in the embodiments shown in FIG. 1, the memory 120 arranged in the electronic device 100 is taken as an example. In other embodiments of the present disclosure, the memory 120 may not be arranged in the electronic device 100. In this case, the memory 120 may be connected to the electronic device 100 through an interface provided by the electronic device 100, and further may be connected to the processor 110 in the electronic device 100.

In some embodiments, the functions of electroencephalogram signal acquisition and near-infrared brain function imaging signal acquisition may be integrated into the electronic device 100. In this case, the electronic device 100 may further include: an electroencephalogram signal acquisition assembly 103 and a near-infrared brain function imaging assembly 104. The electroencephalogram signal acquisition assembly 103 is configured to acquire an electroencephalogram signal of a user, and the near-infrared brain function imaging assembly 104 is configured to acquire near-infrared brain function imaging information of the user.

In some embodiments, the electroencephalogram signal acquisition assembly 103 and the near-infrared brain function imaging assembly 104 may also be implemented by a multimodal brain function imaging assembly having both the functions of electroencephalogram signal acquisition and near-infrared brain function imaging signal acquisition.

Hereinafter, the method for processing the workload state based on multimodal data according to the embodiments of the present disclosure will be described in detail below with reference to the above system structure and the structure of the electronic device.

FIG. 2 is a schematic flowchart of a method for processing a workload state based on multimodal data according to an embodiment of the present disclosure, and the method may be executed by the electronic device shown in FIG. 1A and FIG. 1B.

As shown in FIG. 2, the method may include steps of 201, 202, and 203.

In step 201, state information of a first user is acquired.

In an example, the state information may include at least two of: physiological information, eye movement information, electroencephalogram information, brain function imaging information, motion capture information, spatiotemporal acquisition information, behavior acquisition information, and facial expression and state information.

The physiological information may include an electrocardiogram signal and other signals related to the physiology of the first user. The above information may be detected using a physiological information detection device such as an electrocardiogram detector.

The motion capture information is information obtained by capturing a motion of the first user, which may be obtained by capturing an image of the first user through a camera and detecting based on the captured image.

The spatiotemporal acquisition information may be obtained by encoding video information, temporal information, and spatial information of a scenario in which the first user is located.

The behavior acquisition information is information obtained by acquiring a behavior of the first user, and specifically may be obtained by capturing a video image of the first user through a camera and detecting the behavior of the first user based on the captured image.

The facial expression and state information is information obtained by acquiring the facial expression and state of the first user, and specifically may be obtained by capturing a facial image of the first user through a camera and detecting based on the facial image.

In some embodiments, in this step, the first electroencephalogram signal and the first near-infrared brain function imaging signal of the first user may be acquired.

In an example, if the method shown in FIG. 2 is applied to the system shown in FIG. 1A, the electronic device may control the electroencephalogram signal acquisition device to acquire the first electroencephalogram signal of the first user, and control the near-infrared brain function imaging device to acquire the first near-infrared brain function imaging information of the first user.

In another example, if the method shown in FIG. 2 is applied to the electronic device shown in FIG. 1B, the electronic device may control the electroencephalogram signal acquisition assembly to acquire the first electroencephalogram signal of the first user, and control the near-infrared brain function imaging assembly to acquire the first near-infrared brain function imaging information of the first user.

In an example, the first electroencephalogram signal may be a whole-brain electroencephalogram signal, which refers to an electroencephalogram signal obtained by detecting the entire brain.

Near-infrared brain function imaging is a latest-generation brain function detection technology based on optical principles. The first near-infrared brain function imaging signal in this step is a signal obtained using a near-infrared brain function imaging technology. During cerebral neural activity, intracerebral metabolic changes occur, and cerebral cortex hemodynamics subsequently change accordingly, thereby causing changes in oxygenated hemoglobin (HbO2) and deoxygenated hemoglobin (Hb) in brain tissue. Near-infrared light (650 nm to 900 nm) signals can penetrate human tissue and skull to reach the cerebral cortex layer (to a depth of about 3 cm below the scalp). According to the correlation between optical attenuation and the concentration changes of chromophores (HbO and Hb) in the brain, hemodynamic changes of the cerebral cortex can be detected, and then the cerebral neural activity can be inferred in reverse through a neurovascular coupling rule. The detected signal may be called a near-infrared brain function imaging signal.

In step 202, workload information of the first user is determined according to the acquired state information.

In an example, in this step, state features may be extracted from the acquired state information, and the extracted multimodal features may be subjected to fusion processing to obtain multimodal fusion features. The workload information of the first user may be determined according to the fusion features.

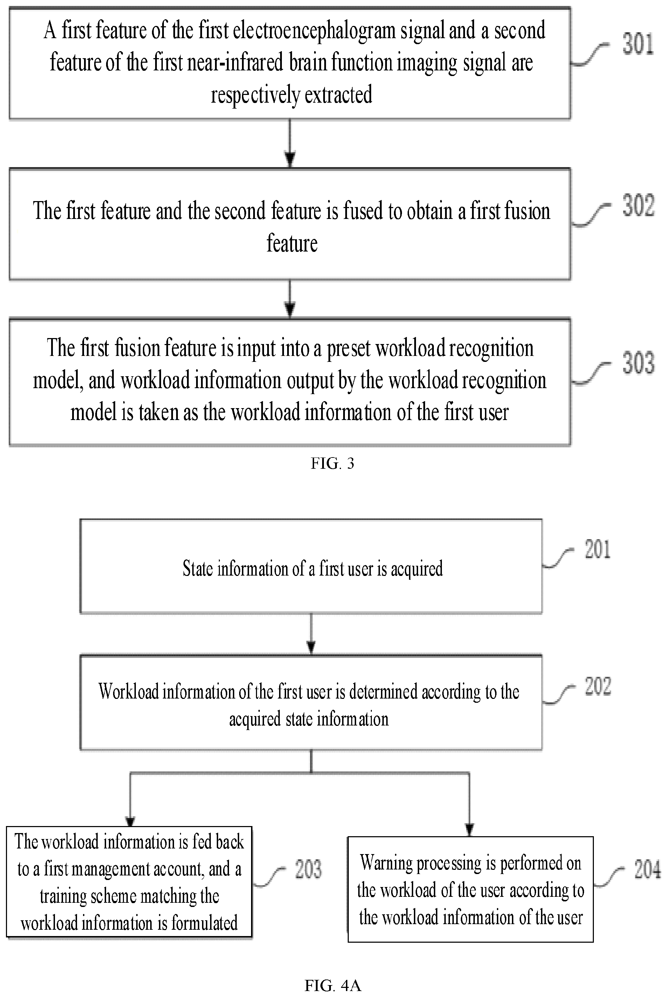

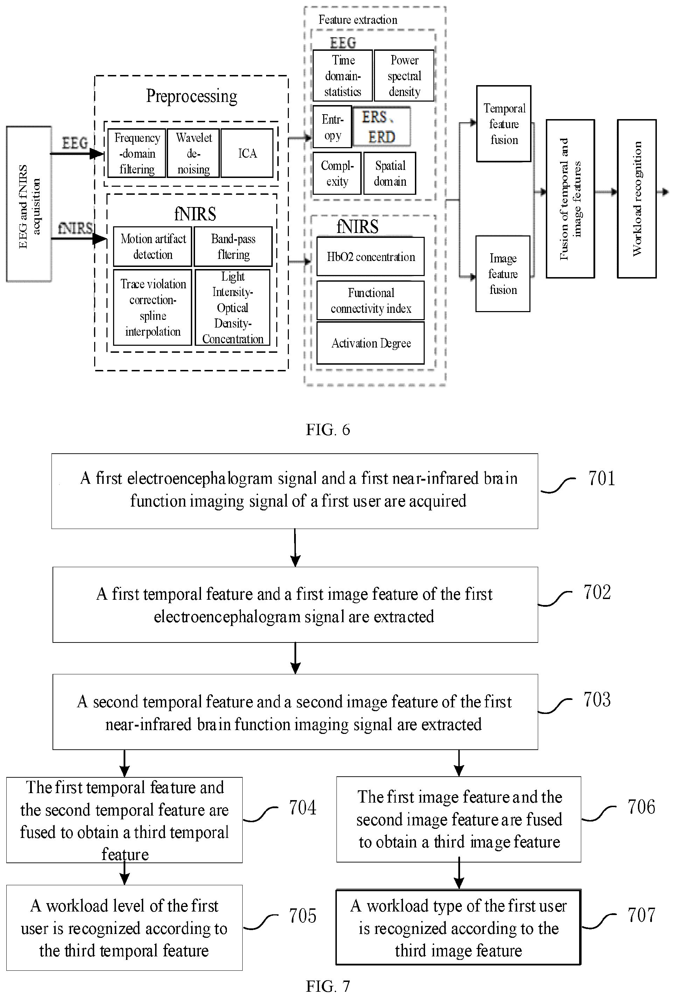

For example, if the state information of the first user acquired in step 201 includes the first electroencephalogram signal and the first near-infrared brain function imaging signal, step 202 may be implemented through the embodiments shown in FIG. 3, or may be implemented through steps 704 to 707 in the embodiments shown in FIG. 7.

As shown in FIG. 3, step 202 may specifically include step 301, step 302, and step 303.

In step 301, a first feature of the first electroencephalogram signal and a second feature of the first near-infrared brain function imaging signal are respectively extracted.

In step 302, the first feature and the second feature is fused to obtain a first fusion feature.

In step 303, the first fusion feature is input into a preset workload recognition model, and workload information output by the workload recognition model is taken as the workload information of the first user.

The following is an exemplary description of possible implementations of the above step 301.

In an example, the first feature of the first electroencephalogram signal may include: a first temporal feature and a first image feature of the first electroencephalogram signal.

The first temporal feature of the first electroencephalogram signal refers to time-related features of the first electroencephalogram signal. In an example, the first temporal feature of the first electroencephalogram signal may include: a time-domain feature, a frequency-domain feature, a time-frequency-domain feature, a nonlinear feature, and/or a brain function connection feature of the first electroencephalogram signal. In some related technologies, the above features may also be referred to as indicators.

In an example, the time-domain feature of the first electroencephalogram signal may include: kurtosis, skewness, mean, standard deviation, event related potential (ERP) latency, and peak value of the ERP of the first electroencephalogram signal.

The meanings and characterization significance of the above time-domain features are shown in Table 1 below.

**Table 1**

| Indicator Name | Description | Significance |
|---|---|---|
| Kurtosis | Kurtosis is a statistic describing the shape of the signal data distribution, and typically calculated using a fourth order central moment. | High kurtosis indicates the presence of sharp peaks or very flat regions in the signal, while low kurtosis indicates a smoother signal. |
| Skewness | Skewness is a statistic describing the degree to which the signal data distribution is skewed to the left or right. | The reflection of the non-uniformity of brain activity |
| Mean | Mean is the arithmetic average of data. | The overall voltage level of the electroencephalogram signal |
| Standard Deviation | Standard deviation is the degree of dispersion of data points from the mean. | The overall stability of the electroencephalogram signal |
| Event related potential latency | Event related potential latency is a time delay of an electroencephalogram response evoked by a specific event or stimulus. | The processing speed of the brain |
| Event related potential amplitude | Amplitude refers to the peak value of a signal or the height from peak to trough of a signal. | The intensity of the cognitive processing induced by a stimulus |

In an example, the time-domain feature of the first electroencephalogram signal may be extracted using a time-domain analysis method for the electroencephalogram signal in the related art. The time-domain analysis method may be, for example, event related potential (ERP) or spatiotemporal modeling.

The frequency-domain feature of the first electroencephalogram signal is used to describe the distribution of information such as energy and phase of the first electroencephalogram signal at frequency points, which can reflect a cognitive state of the user. In an example, the frequency-domain feature of the first electroencephalogram signal may include: energy values of the first electroencephalogram signal in five frequency bands, namely Delta, Theta, Alpha, Beta, and Gamma, as well as brain cognitive function features such as α/β, θ/β, (α+θ)/β, (α+θ)/(α+β), θ/(α+β), and SMR(db). α represents an Alpha wave of the first electroencephalogram signal, β represents a Beta wave of the first electroencephalogram signal, θ represents a Theta wave of the first electroencephalogram signal, and SMR represents a sensorimotor rhythm (SMR) wave of the first electroencephalogram signal.

The meanings and characterization significance of the above frequency bands and frequency-domain features are shown in Table 2 below.

**Table 2**

| Indicator Name | Description | Significance |
|---|---|---|
| Delta | Typically in the range of 0.5 Hz to 4 Hz | Higher Delta values may represent deep sleep states or pathological conditions. |
| Theta | Typically in the range of 4 Hz to 8 Hz | Associated with relaxed states, creative thinking, and attention shifting. Increased Theta waves may suggest inattention or a relaxed state. |
| Alpha | Typically in the range of 8 Hz to 13 Hz | Occurs during rest or eyes-closed quiet sitting, associated with relaxation and resting attention. Reduced Alpha waves may represent increased alertness. |
| Beta | Typically in the range of 13 Hz to 30 Hz | Associated with alertness, cognition, and attention. Increased Beta waves may represent an excited state or cognitive activity. |
| Gamma | Typically above 30 Hz | Associated with higher-order cognition, learning, and information processing. Increased Gamma waves may represent highly cognitive activity. |
| Power Peak | The maximum power value in the spectrum, generally corresponding to a specific frequency band | The power peaks may provide information about dominant frequency characteristics of the electroencephalogram activity. |
| α/β | The ratio of Alpha wave to Beta wave, generally used to explore the balance between alertness and relaxed states | A higher α/β ratio may represent a relaxed state. |
| θ/β | The ratio of Theta wave to Beta wave, used to assess attention and alertness levels | A higher θ/β ratio may represent inattention. |
| (α+θ)/β | The ratio of the sum of Alpha wave and Theta wave to Beta wave. | Providing integrated information about relaxed states and attention. |
| (α+θ)/(α+β) | The ratio of the sum of Alpha wave and Theta wave to the sum of Alpha wave and Beta wave. | Overall balance of electroencephalogram waves |
| θ/(α+β) | The ratio of Theta wave to the sum of Alpha wave and Beta wave. | Assessing the balance between attention and rest states. |
| SMR(db) | Representing sensorimotor rhythm, typically in the range of 12Hz to 15 Hz. | SMR relates to sensory motor integration and control. |

The frequency-domain feature may be obtained by analyzing the first electroencephalogram signal using a relevant frequency-domain analysis method for the electroencephalogram signal, such as Fourier transform, a periodogram method (welch), a multi-window method, and/or an autoregressive model. The specific implementation will not be repeated in the embodiments of the present disclosure.

Fourier transform is a basic frequency-domain analysis method for converting a time-domain signal into a frequency-domain signal. It can decompose a signal into amplitude and phase information of different frequency components. Fourier transform is generally applicable to steady-state signals.

The periodogram method is a method for studying periodic components of a signal, including an autocorrelation function, a cross-correlation function, and a power spectral density function.

The multi-window method involves analyzing spectral characteristics of the signal using different types of window functions, such as a rectangular window, a Hamming window, and a Hanning window. Different window functions are suitable for different applications and signal types.

The autoregressive model is a method for modeling time-series data, generally for estimating frequency components of a signal. The autoregressive model includes an autoregressive (AR) model and an autoregressive-moving average system (ARMA) model.

In an example, the time-frequency-domain feature of the first electroencephalogram signal may include: the power value of the signal at each specific time and frequency point.

The time-frequency-domain features of the first electroencephalogram signal are used to indicate that after the occurrence of a certain stimulus, the corresponding region of interest (ROI) has an increase (Event-Related Synchronization, ERS) or decrease (Event-Related Desynchronization, ERD) in the signal power of a specific frequency band. These features can be obtained by processing the first electroencephalogram signal using a time-frequency-domain analysis method for the electroencephalogram signal, and the time-frequency-domain analysis method includes: short-time Fourier transform, continuous wavelet transform, etc.

Nonlinear features of the first electroencephalogram signal are used to reflect changes in the dynamic characteristics of the brain. In an example, the nonlinear features of the first electroencephalogram signal may include: complexity, various entropy, and/or Lyapunov exponents of the first electroencephalogram signal, etc. The nonlinear features may be obtained by analyzing the first electroencephalogram signal using nonlinear dynamics.

The complexity of the first electroencephalogram signal may be used to measure the information capacity of an electroencephalogram segment, thereby reflecting the potential activity characteristics of neurons and representing the speed at which a new pattern appears in the time series as the length of the series increases. In an example, the complexity of the first electroencephalogram signal may include: Lempel-Ziv complexity, Lempel-Ziv permutation complexity, etc.

The entropy value of the first electroencephalogram signal indicates the degree of disorder of the first electroencephalogram signal. Different entropy algorithms describe the capacity of the information from different perspectives, and a decrease in entropy value means a decrease in the information interaction ability in the brain. The entropy of the first electroencephalogram signal may include time-domain-based Shannon entropy, approximate entropy, sample entropy, and permutation entropy, and the time-frequency-based entropy including wavelet entropy, Hilbert-Huang spectrum entropy, etc.

The maximum Lyapunov exponent of the first electroencephalogram signal can quantitatively describe the average divergence rate of neighboring trajectories in the phase space, and how small initial state disturbances in the system gradually increase over time.

The brain function connection feature of the first electroencephalogram signal is used to evaluate information connectivity between brain regions of the brain. Each part of the brain has its unique function in human behavior, and even the simplest task requires cooperation of multiple brain regions. In an example, the brain function connection feature of the first electroencephalogram signal may include: coherence-based features, phase-synchronization-based features, generalized-ynchronization-based features, Granger-causality-based features, etc.

The first image features of the first electroencephalogram signal refers to a spatially related features of the first electroencephalogram signal. In an example, the first image features of the first electroencephalogram signal may include: a 2D map of the first electroencephalogram signal, and/or a 3D dense connection network.

In an example, the specified time-domain features and/or brain function connection features of the first electroencephalogram signal can be visualized based on a graph theory or an international 10-20 system, and then spatial domain information analysis can be performed based on a common spatial pattern (CSP), to construct a 2D map of the first electroencephalogram signal. The specific method may be implemented through relevant technologies, which is not repeated herein.

In an example, a 3D dense connection network may be constructed based on the time-domain feature, the frequency-domain feature, and/or the time-frequency-domain feature of the first electroencephalogram signal. The specific construction method may be completed through relevant technologies, which is not repeated herein.

In an example, the second feature of the first near-infrared brain function imaging signal may include: a second temporal feature and a second image feature of the first near-infrared brain function imaging signal.

The second temporal feature of the first near-infrared brain function imaging signal refers to the time-related features of the first near-infrared brain function imaging signal. In an example, the second temporal feature of the first near-infrared brain function imaging signal may include: concentrations of oxygenated and deoxygenated hemoglobin, a β-index of a single channel, and/or a Pearson correlation coefficient between channels.

In an example, the concentrations of oxygenated and deoxygenated hemoglobin of the first near-infrared brain function imaging signal may be calculated based on the modified Beer-Lambert law. The β-index of the single channel of the first near-infrared brain function imaging signal may be determined using an activation degree analysis method based on a general linear model (GLM). The Pearson correlation coefficient between channels of the first near-infrared brain function imaging signal may be calculated using a preset brain network.

In an example, the second image feature of the first near-infrared brain function imaging signal may include: a 3D channel activation map, a correlation matrix formed between channels, and/or a brain network connection map.

In an example, the 3D channel activation map of the first near-infrared brain function imaging signal may be determined based on the GLM. The brain network connection map of the first near-infrared brain function imaging signal may be calculated using methods such as seed correlation analysis, complex brain network connection, and/or small-world characteristics.

The following provides exemplary descriptions of a seed correlation analysis method, a complex brain network connection analysis method, and an analysis method of small-world characteristics.

Seed correlation analysis: the correlation degree between the selected region of interest and other brain regions is calculated according to the correlation theory as the functional connection strength. In the analysis, a general linear model (GLM) may be used to estimate the activation of each channel. The significantly activated channels are determined through a t-test, which are considered as seed points. The time series of the seed points are then correlated with the time series of other channels across the whole brain, and a statistical method is used to determine which channels are closely related to the seed points, thereby obtaining a functional connection map.

Complex brain network: a complex brain network is an analysis method for describing the network structure of interconnections between multiple regions in the brain. First, different regions of the brain are defined as nodes of the network, which may represent specific regions or functions of the brain. Then, the connection strength between these nodes is calculated, and the correlation of time-series data is usually used to measure the association between the nodes. Since the correlation matrix is usually very dense, a threshold needs to be set to remove weaker connections to simplify the network structure. Complex brain network analysis helps to reveal information transfer, collaborative activities, and functional integration among different regions in the brain.

Small-world characteristics: the small-world characteristics are typical features of a complex brain network, which indicates that the network has efficient information transmission and local interconnectivity on the global level. In an example, a small-world network has a shorter average shortest path length, which means that the efficiency of information transmission between nodes is higher. At the same time, the small-world network maintains a high clustering coefficient, reflecting the degree of close connection between nodes. Such structure is similar to a "small world", where information can be quickly transferred to distant nodes, while maintaining tight connections within local regions, thereby helping the brain to play a role in efficient information processing and functional integration.

The electroencephalogram signal can only analyze the information of the cerebral cortex, and there is a certain limitation in exploring the activation of a specific brain region based on the distribution of the functional connection and energy topographic map. According to the embodiments of the present disclosure, the workload of the user is recognized by combining the first near-infrared brain function imaging signal with the first electroencephalogram signal. The first near-infrared brain function imaging signal can provide more accurate spatial information for the activation between brain regions, which enables the method for processing the workload state based on the multimodal data in the embodiments of the present disclosure to more accurately determine the brain region activated by the user such as a pilot in a certain task state.

The following provides an exemplary description of the possible implementation of step 302.

In an example, when the first feature of the first electroencephalogram signal includes a first temporal feature and a first image feature, and the second feature of the first near-infrared brain function imaging signal includes a second temporal feature and a second image feature of the first near-infrared brain function imaging signal, this step may include the following steps.

The first temporal feature and the second temporal feature are fused to obtain a third temporal feature.

The first image feature and the second image feature are fused to obtain a third image feature.

The third temporal feature and the third image feature are fused to obtain the first fusion feature.

In an example, the first temporal feature and the second temporal feature are fused to obtain the third temporal feature, which may include the following steps.

The first temporal feature is converted into a fourth image feature.

The second temporal feature is converted into a fifth image feature.

The fourth image feature and the fifth image feature are fused to obtain a sixth image feature, and the sixth image feature is taken as the third temporal feature.

The first temporal feature may be converted into the fourth image feature using a relevant method for converting a temporal feature into an image feature, which is not limited in the embodiments of the present disclosure.

In an example, the first image feature and the second image feature are fused to obtain the third image feature, which may include the following steps.

Pixel values of pixels corresponding to the first image feature and the second image feature are added or multiplied as pixel values of pixels corresponding to the third image feature, or the first image feature and the second image feature are spliced to obtain the third image feature.

In an example, the fourth image feature and the fifth image feature are fused to obtain the sixth image feature, which may include the following steps.

Pixel values of pixels corresponding to the fourth image feature and the fifth image feature are added or multiplied as pixel values of pixels corresponding to the sixth image feature, or the fourth image feature and the fifth image feature are spliced to obtain the sixth image feature.

In an example, when the third temporal feature is implemented through the sixth image feature, the third temporal feature and the third image feature are fused to obtain a first fusion feature, which may include the following steps.

Pixel values of pixels corresponding to the sixth image feature and the third image feature are added or multiplied as pixel values of pixels corresponding to the first fusion feature, or the sixth image feature and the third image feature are spliced to obtain a seventh image feature as the first fusion feature.

The following provides an exemplary description of the possible implementation of step 303.

In an example, the workload information may include: a workload level and/or a workload type.

The workload level is used to characterize the magnitude of the workload of the user, and may include at least two levels. In an example, the workload level may be divided into three levels: high, medium, and low.

The workload type is used to characterize a type of the workload of the user. For example, the workload type may include: visual, auditory, and/or attention. In an example, the attention type may include fatigue, distraction, concentration, etc.

Taking the workload information including workload level and workload type as an example, the workload recognition model may output a workload type with relatively high workload under each workload level when outputting the corresponding workload level, or may output a workload type with relatively high workload under the workload level only when outputting the workload level of a specified level.

In one example, assuming that the workload level includes three levels: high, medium and low, and the workload type includes: visual, auditory, and attention, the workload recognition model outputs both the workload level and the workload type. For example, if the output is (low, auditory), it may indicate that the workload level is low, and the workload of the auditory type is relatively high; or if the output is (medium, visual), it may indicate that the workload level is medium, and the workload of the visual type is relatively high under the medium level.

In another example, assuming that the workload level includes three levels: high, medium, and low, and the workload type includes: visual, auditory, and attention, the workload recognition model may only output the workload level when recognizing that the workload level is low. When recognizing that the workload level is medium or high, the workload recognition model outputs the workload level and workload type, such as (medium, visual), thereby indicating that the workload level is medium, and the workload of the visual type is relatively high under the medium level.

It may be understood that, when the workload recognition model outputs the workload level and the workload type, the number of output workload types may be one or more, which is not limited in the embodiments of the present disclosure.

In step 203, the workload information is fed back to a first management account, and a training scheme matching the workload information is formulated.

In an example, in this step, the workload information may be directly sent to the first management account, or a report in a preset format may be generated according to the workload information and then sent to the first management account.

In an example, in some embodiments, training schemes corresponding to different workload information may be preset. In this step, the training scheme corresponding to the workload information may be directly obtained, thereby implementing the formulation of a training scheme matching the workload information.

In other embodiments, if the workload information includes the workload level and the workload type, training schemes corresponding to different workload levels and training schemes corresponding to different workload types may be respectively preset. When the training scheme matching the workload information is formulated in this step, the training scheme corresponding to the workload level in the workload information and the training scheme corresponding to the workload type in the workload information may be respectively obtained, and the two training schemes are combined to obtain the training scheme matching the workload information. The specific combination method of the above two training schemes may be implemented through a relevant method, which is not limited in the embodiments of the present disclosure.

By formulating a training scheme matching the workload information of the first user, the training scheme of the first user can meet the workload requirements of the first user, thereby improving the training efficiency and the training safety of the first user.

In the method shown in FIG. 2, the workload information of the first user is determined based on the multimodal state information of the user, thereby recognizing the workload of the user. In addition, the workload information is fed back to the first management account and the training scheme matching the workload information is formulated, so that the training scheme can be adapted to the workload information of the first user, thereby ensuring the scientificity and safety of the training of the first user.

FIG. 4A is another schematic flowchart of a method for recognizing workload information based on multimodal data according to an embodiment of the present disclosure. As shown in FIG. 4A, after step 202 shown in FIG. 2, the method may further include the following step 204.

In step 204, warning processing is performed on the workload of the user according to the workload information of the user.

The execution order between step 204 and step 203 is not limited in the embodiments of the present disclosure.

In an example, this step may specifically include: when the workload level in the workload information is not lower than the preset level and the workload type includes the first type, a warning is performed on the workload type of the first type; and/or when the workload level in the workload information is not lower than the preset level and the workload type includes the second type, the proportion of the second type among multiple workload information obtained within a preset first duration is determined, and when the proportion is not lower than a preset proportion threshold, a warning is performed on the workload type of the second type.

For example, the preset level may be medium, and the first type may be distraction. Then, when the workload level in the workload information is medium or high, and the workload type is distraction, a warning is performed on the distraction workload type.

For another example, the preset level is medium, the second type is fatigue, the first duration is 5 seconds, and the preset proportion threshold is 70%. As a result, when multiple workload information of the user is obtained within 5 seconds, if more than 70% of the workload information has a workload level higher than medium and the workload type includes fatigue, a warning is performed on the fatigue workload type.

It may be understood that the specific warning manner for warning may be text, sound, and/or light, which is not limited in the embodiments of the present disclosure.

According to the embodiments of the present disclosure, warning processing may be performed based on different workload information, thereby improving the working efficiency of the user and ensuring the work safety of the user.

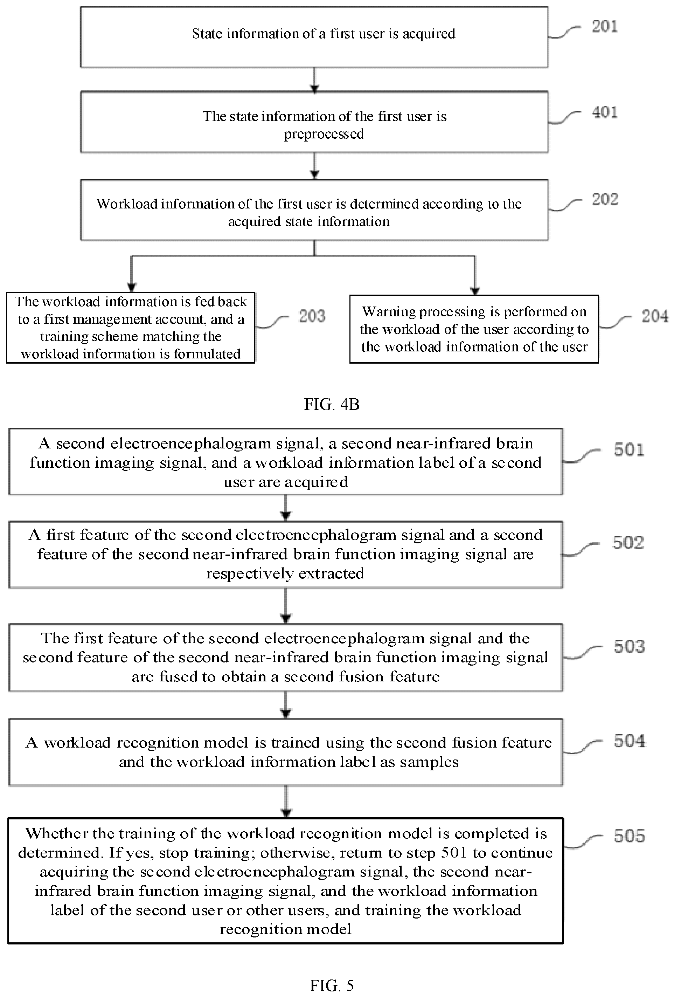

In another embodiment of the method for recognizing workload information based on multimodal data according to the embodiments of the present disclosure, a preprocessing step may be further included between step 201 and step 202 in the above embodiments. As shown in FIG. 4B, taking the preprocessing step shown in step 401 being performed between step 201 and step 202 of the method shown in FIG. 4A as an example.

In step 401, the state information of the first user is preprocessed.

Accordingly, in step 202, the workload information of the first user may be determined based on the preprocessed state information.

The preprocessing may specifically include: noise reduction processing, physiological artifact removal, and/or abnormal value detection, etc.

In an example, if the state information of the first user includes the first electroencephalogram signal and the first near-infrared brain function imaging signal, this step may specifically include that the first electroencephalogram signal and the first near-infrared brain function imaging signal are respectively preprocessed.

Accordingly, in step 301, feature extraction is performed on the preprocessed first electroencephalogram signal and the preprocessed first near-infrared brain function imaging signal.

The preprocessing may specifically include: noise reduction processing, physiological artifact removal, and/or abnormal value detection, etc.

The above preprocessing may be specifically implemented through an end-to-end denoising and artifact removal algorithm such as frequency-domain filtering, independent component analysis (ICA), wavelet denoising, motion artifact detection, band-pass filtering, bad segment and bad channel detection, bad channel interpolation, steady-state evoked response (SSR) short separation regression, and/or a convolutional neural network.

The electroencephalogram data is greatly affected by noise and artifacts. Through the above preprocessing, the first electroencephalogram signal and the first near-infrared brain function imaging signal obtained after processing can be more accurate, so that the subsequently recognized workload result of the user can be more accurate.

The method for training the workload recognition model in the above embodiments is exemplarily described below with reference to FIG. 5. As shown in FIG. 5, the method for training the workload recognition model may include steps of 501, 502, 503, 504, and 505.

In step 501, a second electroencephalogram signal, a second near-infrared brain function imaging signal, and a workload information label of a second user are acquired.

The second user may be any user rather than a certain user. The above first user may also serve as the second user in this step.

In an example, the workload information label is configured to record workload information of the second user when acquiring the second electroencephalogram signal and the second near-infrared brain function imaging signal of the second user.

In step 502, a first feature of the second electroencephalogram signal and a second feature of the second near-infrared brain function imaging signal are respectively extracted.

The implementation of this step may refer to step 301, with the main difference being that the first electroencephalogram signal is replaced with the second electroencephalogram signal in this step, and the first near-infrared brain function imaging signal is replaced with the second near-infrared brain function imaging signal in this step.

In an example, a step of preprocessing the second electroencephalogram signal and the second near-infrared brain function imaging signal may also be included between step 501 and step 502. The implementation of the preprocessing step may refer to step 401, with the main difference being that the first electroencephalogram signal is replaced with the second electroencephalogram signal, and the first near-infrared brain function imaging signal is replaced with the second near-infrared brain function imaging signal, which will not be repeated herein.

In step 503, the first feature of the second electroencephalogram signal and the second feature of the second near-infrared brain function imaging signal are fused to obtain a second fusion feature.

The implementation of this step may refer to step 302, with the main difference being that the first feature of the first electroencephalogram signal is replaced with the first feature of the second electroencephalogram signal in this step, and the second feature of the first near-infrared brain function imaging signal is replaced with the second feature of the second near-infrared brain function imaging signal in this step.

In step 504, a workload recognition model is trained using the second fusion feature and the workload information label as samples.

The workload recognition model in this step is an untrained workload recognition model.

Optionally, the workload recognition model may be implemented through a neural network. In an example, the workload recognition model may include a plurality of network layers. In this step, the workload recognition model is trained using the second fusion feature and the workload information label as samples, which may specifically include the following steps.

The second fusion feature is input into the workload recognition model to obtain an output result of each network layer during a forward propagation process.

A target result of each network layer is determined according to the workload information label.

For each network layer, a similarity between the output result and the target result of the network layer is calculated, and a weight of the network layer is adjusted according to the similarity.

The step of determining the target result of each network layer according to the workload information label may be implemented using relevant technologies, which will not be repeated herein.

The step of calculating the similarity between the output result and the target result of each network layer, and adjusting the weight of the network layer according to the similarity may be implemented using relevant technologies, which will not be repeated herein.

In step 505, whether the training of the workload recognition model is completed is determined. If yes, stop training; otherwise, return to step 501 to continue acquiring the second electroencephalogram signal, the second near-infrared brain function imaging signal, and the workload information label of the second user or other users, and training the workload recognition model.

In this step, a condition for completing the training of the workload recognition model may be preset. The setting of such condition may refer to relevant technologies, and the implementation of determining whether the training of the workload recognition model is completed based on such condition may also refer to relevant technologies, which is not limited in the embodiments of the present disclosure. It may be understood that when it is determined that the training of the workload recognition model is completed, a trained workload recognition model can be obtained, which may serve as the model for recognizing the workload information of the user in step 303.

The method for processing the workload state based on multimodal data according to the embodiments of the present disclosure may be executed by an electronic device having a structure shown in FIG. 1B. The method for training the workload recognition model according to the embodiments of the present disclosure may also be executed by an electronic device having a structure shown in FIG. 1B. It may be understood that the electronic device executing the method for processing the workload state based on multimodal data and the electronic device executing the method for training the workload recognition model may be the same electronic device or different electronic devices.

The method for recognizing workload information based on multimodal data according to the embodiments of the present disclosure is described below with reference to FIG. 6. As shown in FIG. 6, the method includes the following steps.

A first electroencephalogram signal EEG and a first near-infrared brain function imaging signal fNIRS of a user are acquired.

The first electroencephalogram signal EEG is preprocessed, and feature extraction is performed on the preprocessed first electroencephalogram signal EEG to obtain a first temporal feature and a first image feature of the first electroencephalogram signal EEG.

The first near-infrared brain function imaging signal fNIRS is preprocessed, and feature extraction is performed on the preprocessed first near-infrared brain function imaging signal fNIRS to obtain a second temporal feature and a second image feature of the first near-infrared brain function imaging signal fNIRS.

A first temporal feature of the first electroencephalogram signal EEG and a second temporal feature of the first near-infrared brain function imaging signal fNIRS are fused to obtain a third temporal feature.

A first image feature of the first electroencephalogram signal EEG and a second image feature of the first near-infrared brain function imaging signal fNIRS are fused to obtain a third image feature.

The third temporal feature and the third image feature are fused to obtain a first fusion feature.

The first fusion feature is input into a preset workload recognition model to obtain workload information of the user.

A method for recognizing pilot workload based on human-factor intelligence is provided in the following embodiments.

It may be understood that the pilot in the following embodiments may also be extended to users in other work scenarios, such as drivers.

In some embodiments, the method for recognizing pilot workload based on human-factor intelligence described below may serve as a possible implementation of the method for processing the workload state based on multimodal data described above.

Human-factor intelligence combines artificial intelligence (AI) technologies on the basis of human-factor engineering technologies. Human-factor engineering technologies applies human psychological and physiological principles to the engineering and design of a product, a process, and a system. In other words, it is a technology for designing and improving human-machine-environment systems according to human characteristics. Combining this technology with artificial intelligence technologies can enable machines to interact with humans more intelligently.

In the method for recognizing pilot workload based on human-factor intelligence according to the embodiment of the present disclosure, the workload of the user is recognized based on the electroencephalogram data of the user by utilizing the characteristics of the electroencephalogram data, thereby improving the work safety of the user.

FIG. 7 is a schematic flowchart of a method for recognizing pilot workload based on human-factor intelligence according to an embodiment of the present disclosure, and the method may be executed by the electronic device shown in FIG. 1A and FIG. 1B.

As shown in FIG. 7, the method may include steps of 701, 702, 703, 704, 705, 706, and 707.

In step 701, a first electroencephalogram signal and a first near-infrared brain function imaging signal of a first user are acquired.

In an example, if the method shown in FIG. 7 is applied to the system shown in FIG. 1A, the electronic device may control the electroencephalogram signal acquisition device to acquire the first electroencephalogram signal of the first user, and control the near-infrared brain function imaging device to acquire the first near-infrared brain function imaging signal of the first user.

In another example, if the method shown in FIG. 7 is applied to the electronic device shown in FIG. 1B, the electronic device may control the electroencephalogram signal acquisition assembly to acquire the first electroencephalogram signal of the first user, and control the near-infrared brain function imaging assembly to acquire the first near-infrared brain function imaging signal of the first user.

In an example, the first electroencephalogram signal may be a whole-brain electroencephalogram signal, which refers to an electroencephalogram signal obtained by detecting the entire brain.

Near-infrared brain function imaging is a latest-generation brain function detection technology based on optical principles. The first near-infrared brain function imaging signal in this step is a signal obtained using a near-infrared brain function imaging technology.

The first electroencephalogram signal acquired in this step has a relatively high time resolution, while the first near-infrared brain function imaging signal has a relatively high spatial resolution. The former is electrical activity, and the latter is hemodynamic change. The two are complementary to a certain extent. By acquiring the above two signals in the following steps for joint analysis in subsequent steps, the accuracy of user workload detection can be improved.

In step 702, a first temporal feature and a first image feature of the first electroencephalogram signal are extracted.

The first temporal feature of the first electroencephalogram signal refers to time-related features of the first electroencephalogram signal. In an example, the first temporal feature of the first electroencephalogram signal may include: a time-domain feature, a frequency-domain feature, a time-frequency-domain feature, a nonlinear feature, and/or a brain function connection feature of the first electroencephalogram signal. In some related technologies, the above features may also be referred to as indicators.

The specific implementations of the time-domain feature, the frequency-domain feature, the time-frequency-domain feature, the nonlinear feature, and/or the brain function connection feature of the first electroencephalogram signal may refer to the corresponding descriptions in step 301, which will not be repeated herein.

The first image features of the first electroencephalogram signal refers to a spatially related features of the first electroencephalogram signal. In an example, the first image feature of the first electroencephalogram signal may include a brain topographic map, and/or a power spectrum topographic map.

In an example, the brain topographic map and/or the power spectrum topographic map may be obtained using a feature extraction method of a relevant brain function image, which is not limited in the embodiments of the present disclosure.

In step 703, a second temporal feature and a second image feature of the first near-infrared brain function imaging signal are extracted.

The second temporal feature of the first near-infrared brain function imaging signal refers to the time-related features of the first near-infrared brain function imaging signal. In an example, the second temporal feature of the first near-infrared brain function imaging signal may include: concentrations of oxygenated and deoxygenated hemoglobin, a β-index of a single channel, and/or a Pearson correlation coefficient between channels.

In an example, the concentrations of oxygenated and deoxygenated hemoglobin of the first near-infrared brain function imaging signal may be calculated based on the modified Beer-Lambert law. The β-index of the single channel of the first near-infrared brain function imaging signal may be determined using an activation degree analysis method based on a general linear model (GLM). The Pearson correlation coefficient between channels of the first near-infrared brain function imaging signal may be calculated using a preset brain network.

In some embodiments, in this step, brain network maps with different functions may be extracted from the first near-infrared brain function imaging signal as the second image feature.

The human brain includes a plurality of different brain networks, such as a dorsal attention network (DAN), a default mode network (DMN), a sensorimotor Network (SMN), DAN-SMN, DAN-VAN, a ventral attention network (VAN), an auditory attention network (AAN), a frontoparietal network (FPN), a social network (SN), etc. Exemplary descriptions are respectively provided below.

DAN: it is responsible for top-down attention control, helping the brain orient attention externally, such as for motor control or visual search.

DMN: it is related to introspection, self-awareness, individual cognition and social cognition. It is active during rest and weakens when executing tasks.

SMN: it processes information from sensory organs and controls muscle movement, enabling the body to perceive and respond to the environment.

DAN-SMN: it can predict performance during multi-task processing.

DAN-VAN: it is involved in spatial recognition and spatial attention allocation.

VAN: it plays an important role in allocating and controlling attention in an external environment, goal-directed tasks, interference inhibition, and processing unexpected events, and is a bottom-up attention network.

AAN: it plays an important role in processing auditory information and allocating attention to auditory stimuli.

FPN: it includes the prefrontal cortex and parietal lobe, related to advanced cognitive functions and decision-making.

SN: it involves social interaction, emotional processing, and the perception of social emotions. It plays a key role in understanding and processing social information, emotional expression and social emotions.

In this step, the image features (i.e., the brain network map) of the brain network may be extracted from the first near-infrared brain function imaging signal, so that the workload type of the user may be obtained by analyzing the functional connections between the brain networks in subsequent steps, thereby improving the detection accuracy of the workload type.

In an example, the second image feature of the first near-infrared brain function imaging signal may specifically include: a 3D channel activation map, a complex brain network, a default brain network, a correlation heat map, and/or a brain network connection map.

In an example, the 3D channel activation map of the first near-infrared brain function imaging signal may be determined based on the GLM. The brain network connection map of the first near-infrared brain function imaging signal may be calculated using methods such as seed correlation analysis, complex brain network connection, and/or small-world characteristics.

The execution order between step 702 and step 703 is not limited in the embodiments of the present disclosure.

In step 704, the first temporal feature and the second temporal feature are fused to obtain a third temporal feature.

In an example, the fusion processing in this step may be implemented using a relevant temporal feature fusion method, such as a temporal feature weighted fusion method, which is not limited in the embodiments of the present disclosure.

In step 705, a workload level of the first user is recognized according to the third temporal feature.

The workload level is used to characterize the magnitude of the workload of the user, and is a measurement of the overall workload of the user. It may include at least two levels. In an example, the workload level may be divided into three levels: high, medium, and low.

In some embodiments, a load level recognition model may be pre-trained, the input of the model may be a temporal feature of the user, and the input may be a workload level. The method for training the model may refer to the description of FIG. 7, which will not be repeated herein. In an example, this step may specifically include: inputting the third temporal feature into the load level recognition model, and taking the workload level output by the load level recognition model as the workload level of the first user.

In step 706, the first image feature and the second image feature are fused to obtain a third image feature.

In an example, the fusion processing in this step may be implemented using a relevant fusion method, such as a pixel-level fusion method, a feature-level fusion method (for example, a weighted average method, a Bayesian estimation method, a cluster analysis method, etc.), or a decision-level fusion method, which is not limited in the embodiments of the present disclosure.

In step 707, a workload type of the first user is recognized according to the third image feature.

The workload type is used to characterize a type of the workload of the user. For example, the workload type may include: visual, auditory, attention, executive, and/or planning.

In some scenarios, the work of a worker is relatively complex. Taking a pilot flying an aircraft or performing a flight simulation as an example, flying an aircraft is essentially a complex continuous tracking task, which requires a bottom-up process to perform multisensory integration on information from the external environment, and also requires a top-down regulatory influence, a strategy and a current intention based on an internal goal of the pilot. Therefore, in a complex work scenario such as the pilot flying aircraft or performing the flight simulation, predicting the workload of the pilot has very important significance and value.

In the embodiments of the present disclosure, the workload type of the user during work (for example, during a pilot flying an aircraft or performing a flight simulation) may be predicted based on relationships between different networks in the brain and different workload types such as visual, auditory, attention, executive, and/or planning.

In some embodiments, a load type recognition model may be pre-trained, the input of the model may be an image feature of the user, and the input may be a workload type. The method for training the model may refer to the description of FIG. 6, which will not be repeated herein. In an example, this step may specifically include: inputting the third image feature into the load type recognition model, and taking the workload type output by the load type recognition model as the workload type of the first user.

It may be understood that when the load type recognition model outputs the workload type, the number of the output workload types may be one or more, which is not limited in the embodiments of the present disclosure.

Referring to FIG. 7, the execution order between step 704 to step 705 and step 706 to step 707 is not limited.

The electroencephalogram signal may be used to analyze the information of the cerebral cortex, and there is a certain limitation in exploring the activation of a specific brain region based on the distribution of the functional connection and energy topographic map. According to the embodiments of the present disclosure, the workload type of the user is recognized by combining the first near-infrared brain function imaging signal with the first electroencephalogram signal. The first near-infrared brain function imaging signal can provide more accurate spatial information for the activation of a brain region and the activation between brain regions, which enables the method for recognizing pilot workload based on human-factor intelligence of the present disclosure to more accurately determine the brain regions activated by the user, such as a pilot, under a specific task state, thereby more accurately determining the workload type of the user, such as a pilot.

According to the method shown in FIG. 7, the first temporal feature and the first image feature of the first electroencephalogram signal of the first user are respectively extracted, and the second temporal feature and the second image feature of the first near-infrared brain function imaging signal of the first user are respectively extracted. The workload level of the first user is recognized according to the third temporal feature obtained by fusing the first temporal feature and the second temporal feature, and the workload type of the first user is recognized according to the third image feature obtained by fusing the first image feature and the second image feature, thereby realizing recognition of the workload of the user.

In some embodiments, before extracting the first temporal feature and the first image feature in step 702, the first electroencephalogram signal may be preprocessed.

Accordingly, in step 702, the first temporal feature and the first image feature may be extracted from the preprocessed first electroencephalogram signal.

In some embodiments, before extracting the second temporal feature and the second image feature in step 703, the first near-infrared brain function imaging signal may be preprocessed.

Accordingly, in step 703, the second temporal feature and the second image feature may be extracted from the preprocessed first near-infrared brain function imaging signal.

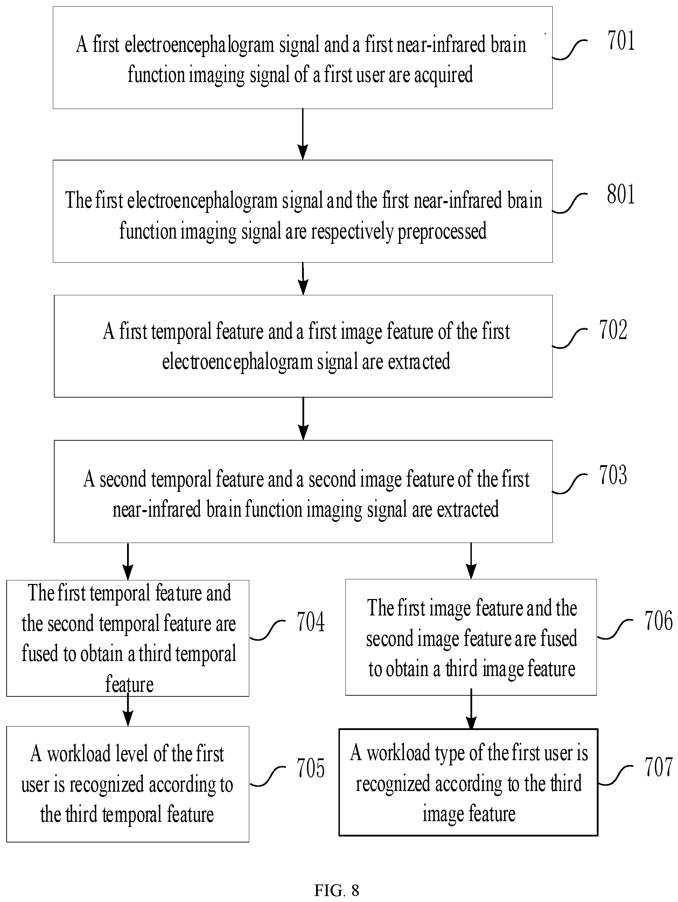

For example, in FIG. 8, step 801 is added before step 702 and step 703 of the method shown in FIG. 7.

In step 801, the first electroencephalogram signal and the first near-infrared brain function imaging signal are respectively preprocessed.

The preprocessing may specifically include: noise reduction processing, physiological artifact removal, and/or abnormal value detection, etc.

The above preprocessing may be specifically implemented through an end-to-end denoising and artifact removal algorithm such as frequency-domain filtering, ICA analysis, wavelet denoising, motion artifact detection, band-pass filtering, bad segment and bad channel detection, bad channel interpolation, steady-state evoked response (SSR) short separation regression, and/or a convolutional neural network.

The electroencephalogram data is greatly affected by noise and artifacts. Through the above preprocessing, the first electroencephalogram signal and the first near-infrared brain function imaging signal obtained after processing can be more accurate, so that the subsequently recognized workload result of the user can be more accurate.

In some embodiments, a warning processing step may be further included after step 705 and step 707 in the above embodiments.

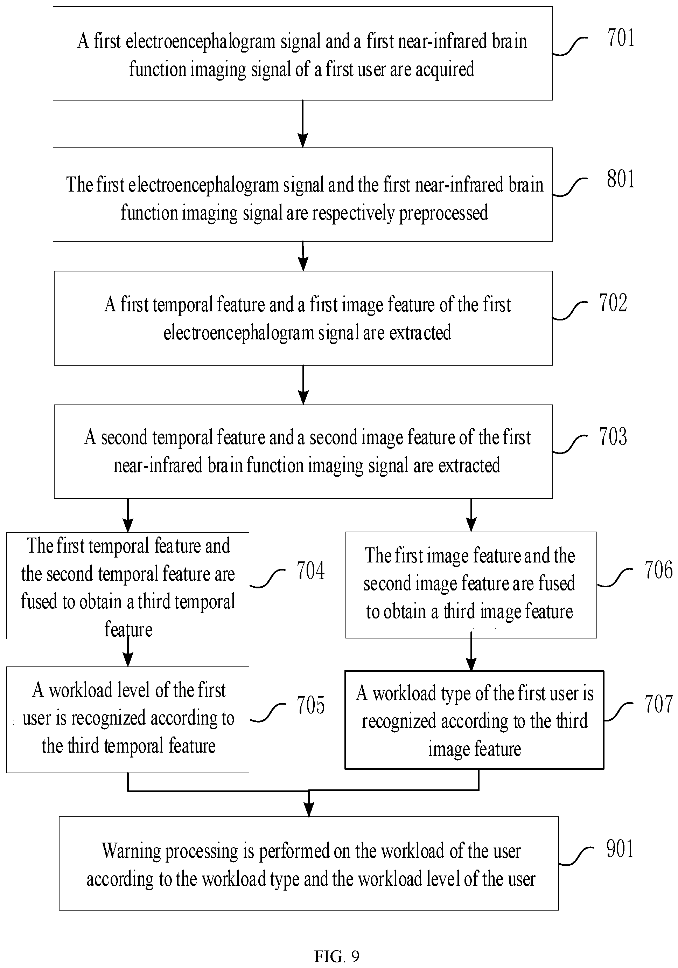

FIG. 9 is another schematic flowchart of a method for recognizing pilot workload based on human-factor intelligence according to an embodiment of the present disclosure, taking the addition of step 901 after step 705 and step 707 shown in FIG. 8 as an example.

In step 901, warning processing is performed on the workload of the user according to the workload type and the workload level of the user.

In an example, this step may specifically include: when the workload level is not lower than a preset level and the workload type includes the first type, performing a warning on the workload type of the first type; and/or when the workload level is not lower than the preset level and the workload type includes the second type, determining the proportion of the second type among multiple workload type obtained within a preset first duration, and when the proportion is not lower than a preset proportion threshold, performing a warning on the workload type of the second type.

For example, the preset level may be medium, and the first type may be visual. Then, when the workload level is medium or high and the workload type is visual, a warning is performed on the visual workload type.

For another example, the preset level is medium, the second type is visual, the first duration is 5 seconds, and the preset proportion threshold is 70%. As a result, when multiple workload types of the user are obtained within 5 seconds, if more than 70% of the workload types have a workload level higher than medium and the workload type includes visual, a warning is performed on the visual workload type.

It may be understood that the specific warning manner for warning may be text, sound, and/or light, which is not limited in the embodiments of the present disclosure.

The embodiments of the present disclosure may be applied to a scenario of a pilot flying aircraft or performing flight simulations. A warning may be provided when the workload of the pilot of a certain type is relatively high, so as to ensure the work safety of the pilot.

According to the embodiments of the present disclosure, warning processing may be performed based on different workload types and workload levels, thereby improving the working efficiency of the user and ensuring the work safety of the user.

In some embodiments, a training scheme formulation step may further be included after step 705 and step 707 in the above embodiments.

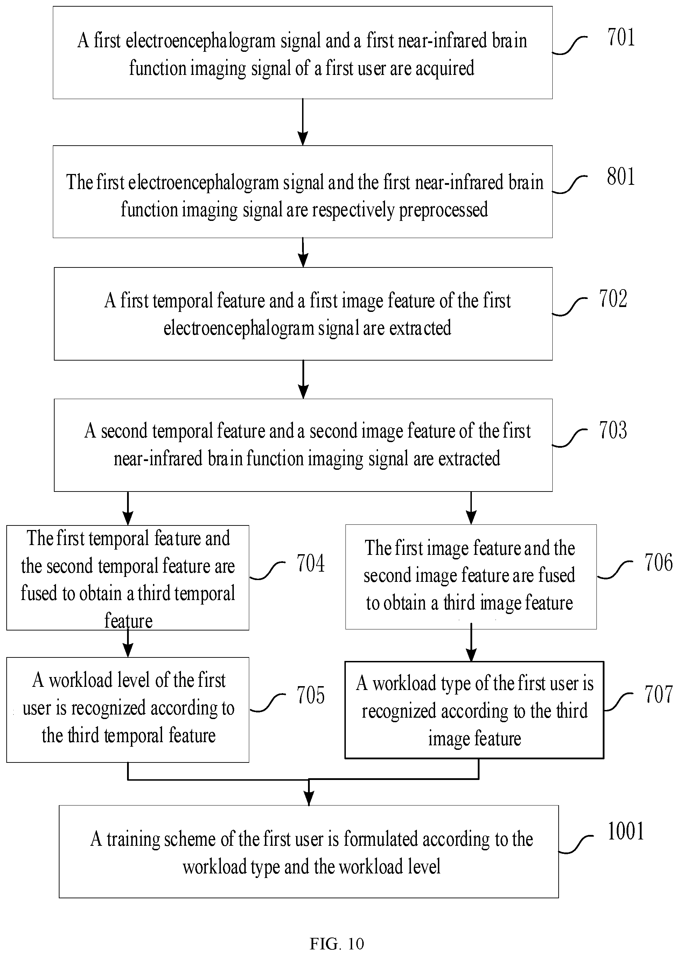

FIG. 10 is another schematic flowchart of a method for recognizing pilot workload based on human-factor intelligence according to an embodiment of the present disclosure. As shown in FIG. 10, the method takes the addition of step 1001 after step 705 and step 707 shown in FIG. 8 as an example.

In step 1001, a training scheme of the first user is formulated according to the workload type and the workload level.

In an example, in some embodiments, training schemes corresponding to combinations of different workload types and workload levels may be preset. In this step, the training scheme corresponding to the workload type and the workload level may be directly obtained, thereby formulating a training scheme matching the workload type and the workload level.

In other embodiments, training schemes corresponding to different workload levels and training schemes corresponding to different workload types may be respectively preset. In this step, the training scheme corresponding to the workload level of the first user and the training scheme corresponding to the workload type of the first user may be respectively obtained, and the two training schemes may be combined to obtain a training scheme matching the workload type and the workload level. The specific combination method of the above two training schemes may be implemented through a relevant method, which is not limited in the embodiments of the present disclosure.

By formulating a training scheme matching the workload type and the workload level of the first user, the training scheme of the first user can adapt to the workload requirements of the first user, thereby improving the training efficiency and the training safety of the first user. The embodiments of the present disclosure can be applied, for example, to pilot flight simulation scenarios, so as to formulate the training scheme for the workload level and the workload type of the pilot during actual flight or flight simulation, thereby improving the training effectiveness and the safety of the pilot.

According to the method, the training scheme of the first user is formulated according to the workload type and the workload level, so that the training scheme can be adapted to the workload type and the workload level of the first user, thereby ensuring the scientificity and safety of the training of the first user.

In some embodiments, other workload-based processing may also be performed according to the workload type and the workload level of the user, such as cockpit optimization, device evaluation, etc., which will not be listed one by one in the embodiments of the present disclosure.

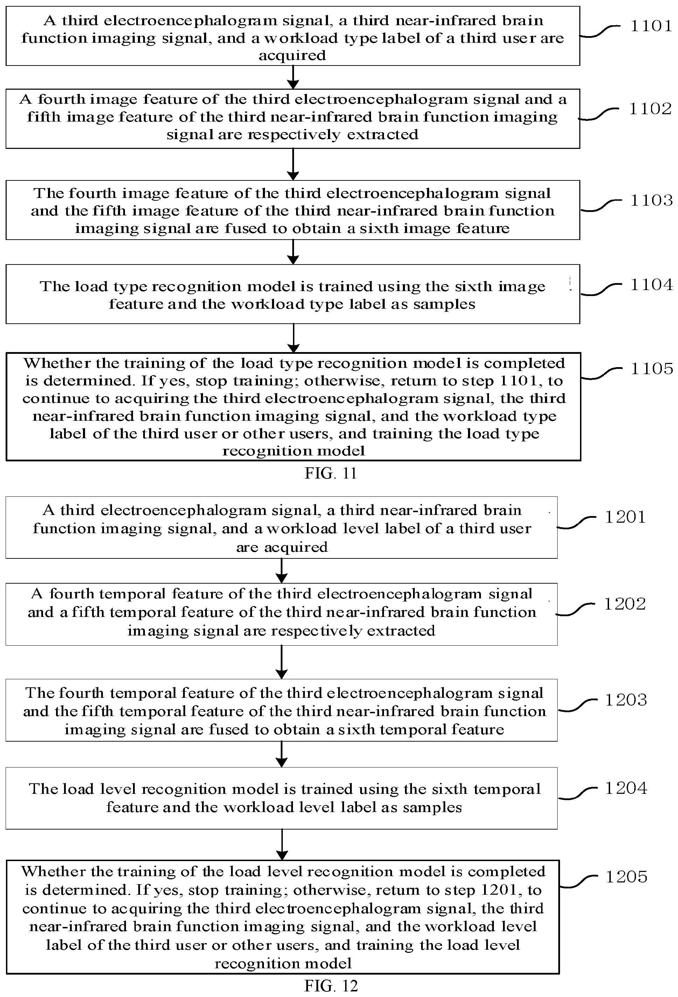

The method for training the load type recognition model in the above embodiments is exemplarily described below with reference to FIG. 11. As shown in FIG. 11, the method for training the load type recognition model may include steps of 1101, 1102, 1103, 1104, and 1105.

In step 1101, a third electroencephalogram signal, a third near-infrared brain function imaging signal, and a workload type label of a third user are acquired.

The third user may be any user rather than a certain user. The aforementioned first user and second user may also serve as the third user in this step.

In an example, the workload type label is configured to record the workload type of the third user when acquiring the third electroencephalogram signal and the third near-infrared brain function imaging signal of the third user.

In step 1102, a fourth image feature of the third electroencephalogram signal and a fifth image feature of the third near-infrared brain function imaging signal are respectively extracted.

The implementation of this step may refer to step 702 and step 703, with the main difference being that the first electroencephalogram signal is replaced with the third electroencephalogram signal in this step, and the first near-infrared brain function imaging signal is replaced with the third near-infrared brain function imaging signal in this step.

In an example, a step of preprocessing the third electroencephalogram signal and/or the third near-infrared brain function imaging signal may also be included between step 1101 and step 1102. The implementation of the preprocessing step may refer to the foregoing corresponding description of the preprocessing step, with the main difference being that the first electroencephalogram signal is replaced with the third electroencephalogram signal, and the first near-infrared brain function imaging signal is replaced with the third near-infrared brain function imaging signal, which will not be repeated herein.

In step 1103, the fourth image feature of the third electroencephalogram signal and the fifth image feature of the third near-infrared brain function imaging signal are fused to obtain a sixth image feature.

The implementation of this step may refer to step 706, with the main difference being that the first image feature of the first electroencephalogram signal is replaced with the fourth image feature of the third electroencephalogram signal in this step, and the second image feature of the first near-infrared brain function imaging signal is replaced with the fifth image feature of the third near-infrared brain function imaging signal in this step.

In step 1104, the load type recognition model is trained using the sixth image feature and the workload type label as samples.

The load type recognition model in this step is an untrained load type recognition model.

Optionally, the load type recognition model may be implemented through a neural network. In an example, the load type recognition model may include a plurality of network layers. In this step, training the load type recognition model using the sixth image feature and the workload type label as samples may specifically include the following steps.

The sixth image feature is input into the load type recognition model to obtain an output result of each network layer during a forward propagation process.

A target result of each network layer is determined according to the workload type label.

For each network layer, a similarity between the output result and the target result of the network layer is calculated, and a weight of the network layer is adjusted according to the similarity.

The step of determining the target result of each network layer according to the workload type label may be implemented using relevant technologies, which will not be repeated herein.

The step of calculating the similarity between the output result and the target result of each network layer, and adjusting the weight of the network layer according to the similarity may be implemented using relevant technologies, which will not be repeated herein.

In step 1105, whether the training of the load type recognition model is completed is determined. If yes, stop training; otherwise, return to step 1101, to continue to acquiring the third electroencephalogram signal, the third near-infrared brain function imaging signal, and the workload type label of the third user or other users, and training the load type recognition model.

In this step, a condition for completing the training of the load type recognition model may be preset. The setting of such condition may refer to relevant technologies, and the implementation of determining whether the training of the load type recognition model is completed based on such condition may also refer to relevant technologies, which is not limited in the embodiments of the present disclosure. It may be understood that when it is determined that the training of the load type recognition model is completed, a trained load type recognition model can be obtained, which may serve as the model for recognizing the workload type of the user in step 707.

The method for recognizing pilot workload based on human-factor intelligence according to the embodiments of the present disclosure may be executed by an electronic device having a structure shown in FIG. 1B. The method for training the load type recognition model according to the embodiments of the present disclosure may also be executed by an electronic device having a structure shown in FIG. 1B. It may be understood that the electronic device executing the method for recognizing pilot workload based on human-factor intelligence and the electronic device executing the method for training the load type recognition model may be the same electronic device or different electronic devices.

The method for training the load level recognition model in the above embodiments is exemplarily described below with reference to FIG. 12. As shown in FIG. 12, the method for training the load level recognition model may include steps of 1201, 1202, 1203, 1204, and 1205.

In step 1201, a third electroencephalogram signal, a third near-infrared brain function imaging signal, and a workload level label of a third user are acquired.

The third user may be any user rather than a certain user. The aforementioned first user and second user may also serve as the third user in this step.

In an example, the workload level label is configured to record the workload level of the third user when acquiring the third electroencephalogram signal and the third near-infrared brain function imaging signal of the third user.

In step 1202, a fourth temporal feature of the third electroencephalogram signal and a fifth temporal feature of the third near-infrared brain function imaging signal are respectively extracted.

The implementation of this step may refer to step 702 and step 703, with the main difference being that the first electroencephalogram signal is replaced with the third electroencephalogram signal in this step, and the first near-infrared brain function imaging signal is replaced with the third near-infrared brain function imaging signal in this step.

In an example, a step of preprocessing the third electroencephalogram signal and/or the third near-infrared brain function imaging signal may also be included between step 1201 and step 1202. The implementation of the preprocessing step may refer to the foregoing corresponding description of the preprocessing step, with the main difference being that the first electroencephalogram signal is replaced with the third electroencephalogram signal, and the first near-infrared brain function imaging signal is replaced with the third near-infrared brain function imaging signal, which will not be repeated herein.

In step 1203, the fourth temporal feature of the third electroencephalogram signal and the fifth temporal feature of the third near-infrared brain function imaging signal are fused to obtain a sixth temporal feature.

The implementation of this step may refer to step 704, with the main difference being that the first temporal feature of the first electroencephalogram signal is replaced with the fourth temporal feature of the third electroencephalogram signal in this step, and the second temporal feature of the first near-infrared brain function imaging signal is replaced with the fifth temporal feature of the third near-infrared brain function imaging signal in this step.

In step 1204, the load level recognition model is trained using the sixth temporal feature and the workload level label as samples.

The load level recognition model in this step is an untrained load level recognition model.

Optionally, the load level recognition model may be implemented through a neural network. In an example, the load level recognition model may include a plurality of network layers. In this step, training the load level recognition model using the sixth temporal feature and the workload level label as samples may specifically include the following steps.

The sixth temporal feature is input into the load level recognition model to obtain an output result of each network layer during a forward propagation process.

A target result of each network layer is determined according to the workload level label.

For each network layer, a similarity between the output result and the target result of the network layer is calculated, and a weight of the network layer is adjusted according to the similarity.

The step of determining the target result of each network layer according to the workload level label may be implemented using relevant technologies, which will not be repeated herein.

The step of calculating the similarity between the output result and the target result of each network layer, and adjusting the weight of the network layer according to the similarity may be implemented using relevant technologies, which will not be repeated herein.

In step 1205, whether the training of the load level recognition model is completed is determined. If yes, stop training; otherwise, return to step 1201, to continue to acquiring the third electroencephalogram signal, the third near-infrared brain function imaging signal, and the workload level label of the third user or other users, and training the load level recognition model.

In this step, a condition for completing the training of the load level recognition model may be preset. The setting of such condition may refer to relevant technologies, and the implementation of determining whether the training of the load level recognition model is completed based on such condition may also refer to relevant technologies, which is not limited in the embodiments of the present disclosure. It may be understood that when it is determined that the training of the load level recognition model is completed, a trained load level recognition model can be obtained, which may serve as the model for recognizing the workload level of the user in step 1205.

The method for recognizing pilot workload based on human-factor intelligence according to the embodiments of the present disclosure may be executed by an electronic device having a structure shown in FIG. 1B. The method for training the load level recognition model according to the embodiments of the present disclosure may also be executed by an electronic device having a structure shown in FIG. 1B. It may be understood that the electronic device executing the method for recognizing pilot workload based on human-factor intelligence and the electronic device executing the method for training the load level recognition model may be the same electronic device or different electronic devices.

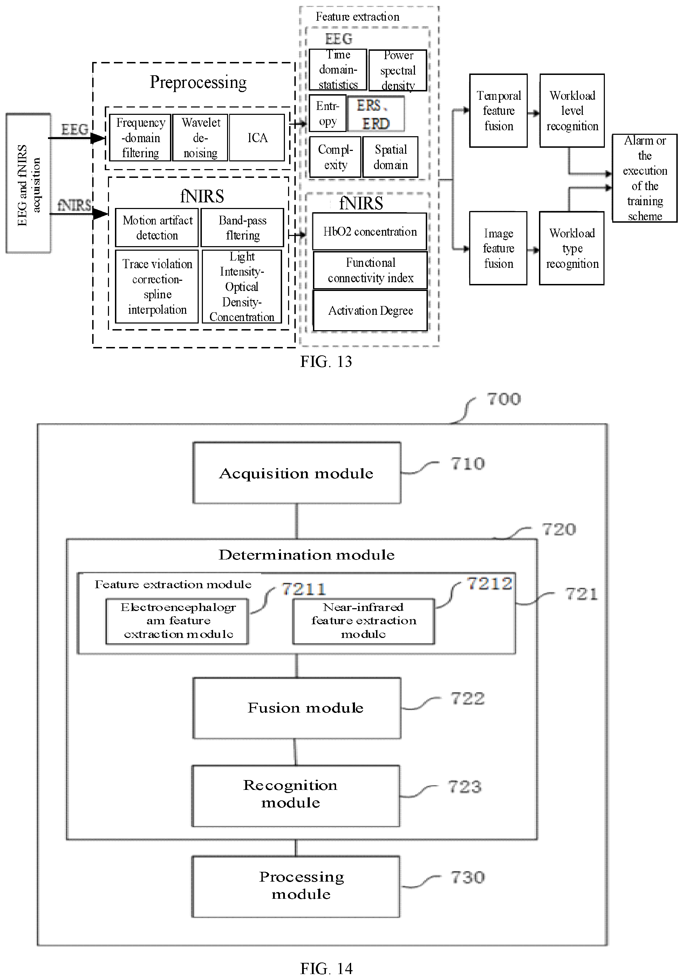

The method for recognizing pilot workload based on human-factor intelligence according to the embodiments of the present disclosure is described below with reference to FIG. 13. As shown in FIG. 6, the method includes the following steps.

A first electroencephalogram signal EEG and a first near-infrared brain function imaging signal fNIRS of a user are acquired.

The first electroencephalogram signal EEG is preprocessed, and feature extraction is performed on the preprocessed first electroencephalogram signal EEG to obtain a first temporal feature and a first image feature of the first electroencephalogram signal EEG.

The first near-infrared brain function imaging signal fNIRS is preprocessed, and feature extraction is performed on the preprocessed first near-infrared brain function imaging signal fNIRS to obtain a second temporal feature and a second image feature of the first near-infrared brain function imaging signal fNIRS.

A first temporal feature of the first electroencephalogram signal EEG and a second temporal feature of the first near-infrared brain function imaging signal fNIRS are fused to obtain a third temporal feature, and the third temporal feature is input into the load level recognition model to obtain the workload level of the user.

A first image feature of the first electroencephalogram signal EEG and a second image feature of the first near-infrared brain function imaging signal fNIRS are fused to obtain a third image feature, and the third image feature is input into the load type recognition model to obtain the workload type of the user.

Then, warning processing, training scheme formulation and other processing may be performed according to the workload level and the workload type of the user.

FIG. 14 is a schematic structural diagram of an apparatus for recognizing workload information based on multimodal data according to an embodiment of the present disclosure. As shown in FIG. 14, the apparatus 700 may include: an acquisition module 710, a determination module 720, and a processing module 730.

The acquisition module 710 is configured to acquire state information of a first user.

The determination module 720 is configured to determine workload information of the first user according to the acquired state information, the state information includes at least two of: physiological information, eye movement information, electroencephalogram information, brain function imaging information, motion capture information, spatiotemporal acquisition information, behavior acquisition information, and facial expression and state information.

The processing module 730 is configured to feed back the workload information to a first management account, and formulate a training scheme matching the workload information.

In an example, when the state information of the first user acquired by the acquisition module 710 includes a first electroencephalogram signal and a first near-infrared brain function imaging signal of the first user, as shown in FIG. 7, the determination module 720 may include: a feature extraction module 721 and an information determination module (not shown in FIG. 14).

The feature extraction module 721 is configured to respectively extract a first feature of the first electroencephalogram signal and a second feature of the first near-infrared brain function imaging signal.

The information determination module is configured to determine the workload information of the first user according to the first feature and the second feature.

In some embodiments, as shown in FIG. 14, the information determination module may include: a fusion module 722 and a recognition module 723.

The fusion module 722 is configured to fuse the first feature and the second feature to obtain a first fusion feature.

The recognition module 723 is configured to input the first fusion feature into a preset workload recognition model, and take workload information output by the workload recognition model as the workload information of the user.

In other embodiments, the information determination module may include a first fusion module 940, a first recognition module 950, a second fusion module 960, and a second recognition module 970 as described in the following embodiments.

In an example, as shown in FIG. 14, the feature extraction module 721 may specifically include: an electroencephalogram feature extraction module 7211 and a near-infrared feature extraction module 7212.

The electroencephalogram feature extraction module 7211 is configured to extract a first feature of the first electroencephalogram signal.

The near-infrared feature extraction module 7212 is configured to extract a second feature of the first near-infrared brain function imaging signal.

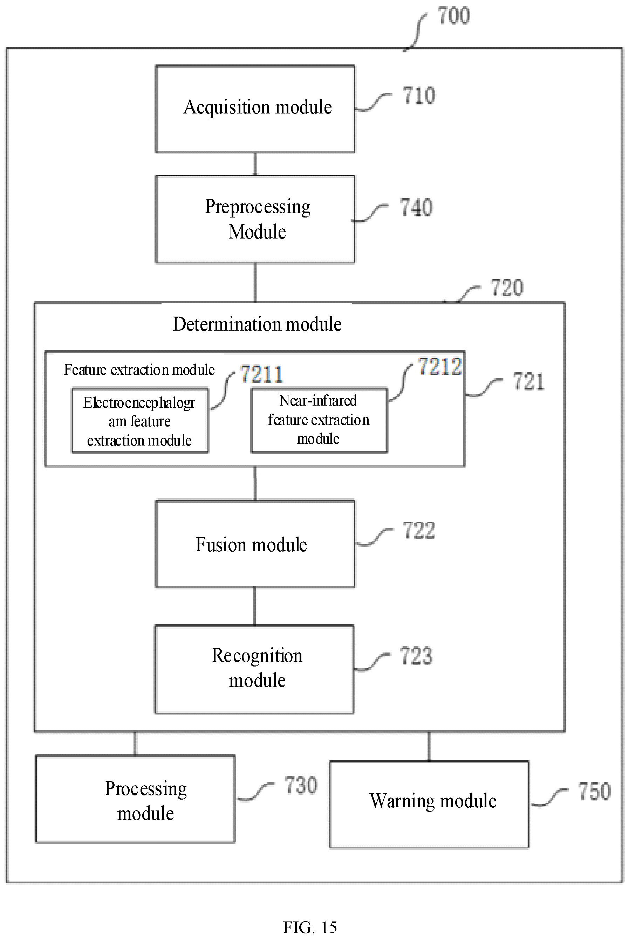

In an example, as shown in FIG. 15, the apparatus 700 may further include a preprocessing module 740 that may be arranged between the acquisition module 710 and the determination module 720.

The preprocessing module 750 is configured to preprocess the state information of the first user.

In an example, when the state information of the first user acquired by the acquisition module 710 includes the first electroencephalogram signal and the first near-infrared brain function imaging signal of the first user, the preprocessing module 750 may respectively preprocess the first electroencephalogram signal and the first near-infrared brain function imaging signal, and send the preprocessed first electroencephalogram signal and the preprocessed first near-infrared brain function imaging signal to the feature extraction module 721.

In an example, as shown in FIG. 15, the apparatus 700 may further include a warning module 750 that is configured to perform warning processing on the workload of the first user according to the workload information of the first user determined by the determination module 720.

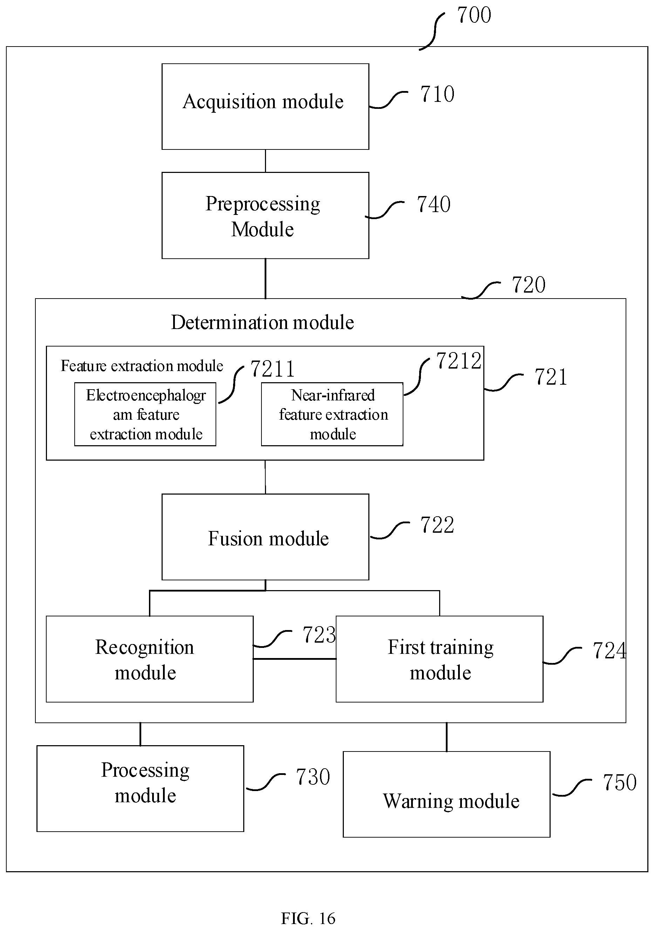

In an example, if the training of the workload recognition model is also performed by the apparatus for recognizing workload, the determination module 720 may further include a first training module 724. FIG. 16 takes the apparatus for recognizing workload shown in FIG. 15 including the first training module 724 as an example.

The acquisition module 710 may be further configured to acquire the second electroencephalogram signal, the second near-infrared brain function imaging signal and a workload information label of the second user.

The preprocessing module 740 is further configured to respectively preprocess the second electroencephalogram signal and the second near-infrared brain function imaging signal, and input the preprocessed second electroencephalogram signal and the preprocessed second near-infrared brain function imaging signal into the feature extraction module 720.

The feature extraction module 721 is further configured to respectively extract the first feature of the second electroencephalogram signal and the second feature of the second near-infrared brain function imaging signal.

The fusion module 722 is further configured to fuse the first feature of the second electroencephalogram signal and the second feature of the second near-infrared brain function imaging signal to obtain a second fusion feature.

The first training module 724 is configured to train the workload recognition model using the second fusion feature and the workload information label as samples, and send the trained workload recognition model to the recognition module 723.

It can be seen that, in the apparatus for recognizing workload shown in FIG. 16, the workload recognition model may first be trained by the acquisition module 710, the preprocessing module 740, the feature extraction module 721, the fusion module 722, and the first training module 724, so as to obtain a trained workload recognition model. The first training module 724 transmits the trained workload recognition model to the recognition module 723. Then, the workload information of a user may be recognized by the acquisition module 710, the preprocessing module 740, the feature extraction module 721, the fusion module 722, and the recognition module 723.

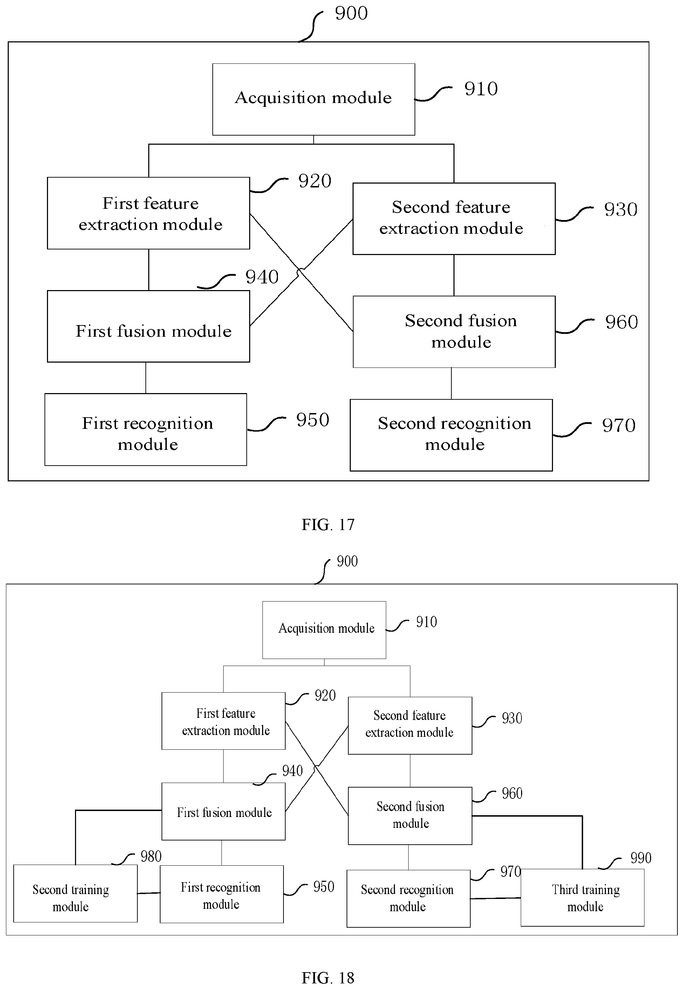

FIG. 17 is a schematic structural diagram of an apparatus for recognizing workload according to an embodiment of the present disclosure. As shown in FIG. 17, the apparatus 900 may include: an acquisition module 910, a first feature extraction module 920, a second feature extraction module 930, a first fusion module 940, a first recognition module 950, a second fusion module 960, and a second recognition module 970.

The acquisition module 910 is configured to acquire the first electroencephalogram signal and the first near-infrared brain function imaging signal of the first user.

The first feature extraction module 920 is configured to extract the first temporal feature and the first image feature of the first electroencephalogram signal.

The second feature extraction module 930 is configured to extract the second temporal feature and the second image feature of the first near-infrared brain function imaging signal.

The first fusion module 940 is configured to fuse the first temporal feature and the second temporal feature to obtain a third temporal feature.

The first recognition module 950 is configured to recognize the workload level of the first user according to the third temporal feature.

The second fusion module 960 is configured to fuse the first image feature and the second image feature to obtain a third image feature.

The second recognition module 970 is configured to recognize the workload type of the first user according to the third image feature.

In some embodiments, as shown in FIG. 10, the apparatus 900 may further include a second training module 980.

The acquisition module 910 is further configured to: acquire the second electroencephalogram signal, the second near-infrared brain function imaging signal, and the workload level label of the second user, and the workload level label is configured to record the workload level of the second user when acquiring the second electroencephalogram signal and the second near-infrared brain function imaging signal of the second user.

The first feature extraction module 920 is further configured to extract a fourth temporal feature of the second electroencephalogram signal.

The second feature extraction module 930 is further configured to extract a fifth temporal feature of the second near-infrared brain function imaging signal.

The first fusion module 940 is further configured to fuse the fourth temporal feature of the second electroencephalogram signal and the fifth temporal feature of the second near-infrared brain function imaging signal to obtain a sixth temporal feature.

The second training module 980 is configured to train the load level recognition model using the sixth temporal feature and the workload level label as samples.

In an example, the second training module may send the trained load level recognition model to the first recognition module 950, so that the first recognition module 950 may recognize the workload level of the user according to the load level recognition model.

In some embodiments, as shown in FIG. 18, the apparatus 900 may further include: a third training module 990.

The acquisition module 910 is further configured to acquire the second electroencephalogram signal, the second near-infrared brain function imaging signal, and the workload type label of the second user, and the workload type label is configured to record the workload type of the second user when acquiring the second electroencephalogram signal and the second near-infrared brain function imaging signal of the second user.

The first feature extraction module 920 is further configured to extract a fourth image feature of the second electroencephalogram signal.

The second feature extraction module 930 is further configured to extract a fifth image feature of the second near-infrared brain function imaging signal.

The second fusion module 960 is further configured to fuse the fourth image feature of the second electroencephalogram signal and the fifth image feature of the second near-infrared brain function imaging signal to obtain a sixth image feature.

The third training module 990 is configured to train the load type recognition model using the sixth image feature and the workload type label as samples.

In an example, the third training module may send the trained load type recognition model to the second recognition module 970, so that the second recognition module 970 may recognize the workload level of the user according to the load type recognition model.

The apparatus provided in the embodiments shown in FIG. 14 to FIG. 18 may be configured to perform the technical solutions in method embodiments of the present disclosure, and for the implementation principle and the technical effect thereof, reference may be further made to related descriptions in the method embodiments.

It should be understood that the division of the modules of the apparatus shown in FIG. 14 to FIG. 18 is merely a division of logical functions. In actual implementation, all or some modules may be integrated into a single physical entity, or may be physically separated. Further, these modules may all be implemented in a form of software called by a processing element; or these modules may all be implemented in a form of hardware; or some modules may be implemented in a form of software called by a processing element, and some modules are implemented in a form of hardware. For example, the acquisition module may be implemented as a separate processing component, or may be integrated into a chip of the electronic device. The other modules are implemented similarly. Further, all or part of these modules may be integrated together, or may be separately implemented. In an implementation process, each step of the above method or each module may be completed through an integrated logic circuit of hardware in a processor element or an instruction in a form of software.

An embodiment of the present disclosure further provides a system for recognizing workload, including the apparatus shown in any one of the above embodiments.

An embodiment of the present disclosure further provides an electronic device, including a processor and a memory, and the processor is configured to implement the method according to the embodiments of the present disclosure.

An embodiment of the present disclosure further provides a system for recognizing workload, including an electronic device, an electroencephalogram signal acquisition device, and a near-infrared brain function imaging device, and the electronic device is configured to implement the method according to the embodiments of the present disclosure.

An embodiment of the present disclosure further provides a computer-readable storage medium on which a computer program is stored. The computer program, when running on a computer, is configured to implement the method provided in the embodiments of the present disclosure.

An embodiment of the present disclosure further provides a computer program product, which includes a computer program. The computer program, when running on a computer, is configured to implement the method according to the embodiments of the present disclosure.

In the embodiments of the present disclosure, "at least one" means one or more, and "a plurality of" means two or more. It should be understood that the term "and/or" describes an associated relationship of an associated object, indicating that there may be three relationships, for example, A and/or B, and may indicate: only A, both A and B, and only B. A and B may be singular or plural. The character "/" herein generally indicates an "or" relationship between associated objects. "At least one of the following" and similar expressions thereof refer to any combination of these items, including any combination of a single item or plural items. Exemplarily, at least one of a, b, and c may represent: a combination of a, b, and c, a combination of a and b, a combination of a and c, a combination of b and c, a, b, and c, where each of a, b, and c may be a single one or multiple.

Those skilled in the art may recognize that the units and algorithm steps described in the embodiments disclosed herein can be implemented using electronic hardware, and a combination of electronic hardware and computer software. Whether these functions are executed in hardware or software depends on the specific application and design constraints of the technical solution. Those skilled in the art may use different methods to implement the described functions for each specific application, but such implementation should not be considered to be beyond a scope of the present disclosure.

Those skilled in the art should clearly understand that, for convenience and conciseness of description, the specific operating process of the above-described system, apparatus and unit can refer to the corresponding process in the above method embodiments , which will not be repeated herein.

In some embodiments provided in the present disclosure, if any function is implemented in the form of the software function unit and is sold or used as an independent product, the function may be stored in the computer-readable storage medium. Based on such understanding, the technical solution of the present disclosure, in essence, the part that contributes to the prior art, or a portion of this technical solution, may be embodied in the form of a software product. The computer software product is stored in the storage medium and includes some instructions for enabling the computer device, which may be a personal computer, a server, or a network device, etc., to execute all or some of the steps of the method described in the embodiments of the present disclosure. The aforementioned storage medium includes: U disk, mobile hard disk, read-only memory (ROM), random access memory (RAM), magnetic disk or optical disk and other media that can store program codes.

The above descriptions are merely specific implementations of the present disclosure. Any person skilled in the art can easily think of changes or substitutions within the technical scope disclosed in the present disclosure, and they should be covered within the protection scope of the present disclosure. The protection scope of the present disclosure should be based on the protection scope of the claims.

## Claims

1. A method for processing a personnel workload state based on multimodal data, comprising:
acquiring state information of a first user;
determining workload information of the first user based on the acquired state information, wherein the state information comprises at least two of: physiological information, eye movement information, electroencephalogram information, brain function imaging information, motion capture information, spatiotemporal acquisition information, behavior acquisition information, and facial expression and state information; and
feeding back the workload information to a first management account, and formulating a training scheme matching the workload information.

2. The method according to claim 1, wherein when the state information of the first user comprises a first electroencephalogram signal and a first near-infrared brain function imaging signal of the first user, said determining workload information of the first user based on the acquired state information comprises:
respectively extracting a first feature of the first electroencephalogram signal and a second feature of the first near-infrared brain function imaging signal; and
determining the workload information of the first user based on the first feature and the second feature.

3. The method according to claim 2, wherein said determining the workload information of the first user based on the first feature and the second feature comprises:
fusing the first feature and the second feature to obtain a first fusion feature; and
inputting the first fusion feature into a preset workload recognition model, and taking workload information output by the workload recognition model as the workload information of the first user.

4. The method according to claim 3, wherein the first feature comprises a first temporal feature and a first image feature, and the second feature comprises a second temporal feature and a second image feature; and
said fusing the first feature and the second feature to obtain a first fusion feature comprises:
fusing the first temporal feature and the second temporal feature to obtain a third temporal feature;
fusing the first image feature and the second image feature to obtain a third image feature; and
fusing the third temporal feature and the third image feature to obtain the first fusion feature.

5. The method according to claim 4, wherein said fusing the first temporal feature and the second temporal feature to obtain a third temporal feature comprises:
converting the first temporal feature into a fourth image feature;
converting the second temporal feature into a fifth image feature; and
fusing the fourth image feature and the fifth image feature to obtain a sixth image feature, and taking the sixth image feature as the third temporal feature.

6. The method according to claim 4 or 5, wherein the first temporal feature comprises: a time-domain feature, a frequency-domain feature, a time-frequency-domain feature, a nonlinear feature, and/or a brain function connection feature, and/or
wherein the first image feature comprises: a two-dimensional (2D) map and/or a three-dimensional (3D) dense connection network, and/or
wherein the second temporal feature comprises: concentrations of oxygenated and deoxygenated hemoglobin, a β-index of a single channel, and/or a Pearson correlation coefficient between channels, and/or
wherein the second image feature comprises: a 3D channel activation map, a correlation matrix formed between channels, and/or a brain network connection map.

7. The method according to any one of claims 1 to 6, wherein the workload information comprises: a workload level and/or a workload type.

8. The method according to claim 2, wherein the first feature comprises a first temporal feature and a first image feature, the second feature comprises a second temporal feature and a second image feature, and the workload information comprises a workload level and a workload type;
said determining the workload information of the first user based on the first feature and the second feature comprises:
fusing the first temporal feature and the second temporal feature to obtain a third temporal feature, and recognizing the workload level of the first user based on the third temporal feature; and
fusing the first image feature and the second image feature to obtain a third image feature, and recognizing the workload type of the first user based on the third image feature.

9. The method according to claim 8, wherein said recognizing the workload level of the first user based on the third temporal feature comprises:
inputting the third temporal feature into a load level recognition model, and taking a workload level output by the load level recognition model as the workload level of the first user.

10. The method according to claim 8 or 9, wherein said recognizing the workload type of the first user based on the third image feature comprises:
inputting the third image feature into a load type recognition model, and taking a workload type output by the load type recognition model as the workload type of the first user.

11. The method according to any one of claims 8 to 10, wherein the first temporal feature comprises: a time-domain feature, a frequency-domain feature, a time-frequency-domain feature, a nonlinear feature, and/or a brain function connection feature, and/or
wherein the first image feature comprises: a brain topographic map and/or a power spectrum topographic map, and/or
wherein the second temporal feature comprises: concentrations of oxygenated and deoxygenated hemoglobin, a β-index of a single channel, and/or a Pearson correlation coefficient between channels, and/or
wherein the second image feature comprises: a 3D channel activation map, a complex brain network, a default brain network, a correlation heat map, and/or a brain network connection map.

12. The method according to any one of claims 7 to 11, wherein the workload type comprises: auditory, visual, attention, executive, and/or planning.

13. The method according to any one of claims 4 to 12, wherein extracting the second image feature of the first near-infrared brain function imaging signal comprises:
extracting brain network maps with different functions from the first near-infrared brain function imaging signal as the second image feature.

14. The method according to any one of claims 7 to 13, further comprising:
performing a warning processing on user workload based on the workload information.

15. The method according to claim 14, wherein said performing a warning processing on user workload based on the workload information comprises:
when the workload level in the workload information is not lower than a preset level, and the workload type in the workload information comprises a first type, alarming for the workload type of the first type.

16. The method according to claim 14, wherein said performing a warning processing on user workload based on the workload information comprises:
when the workload level in the workload information is not lower than a preset level and the workload type in the workload information comprises a second type, determining a proportion of the second type among a plurality of workload information obtained within a preset first duration; and
when the proportion is not lower than a preset proportion threshold, alarming for the workload type of the second type.

17. The method according to any one of claims 1 to 16, wherein said formulating a training scheme matching the workload information comprises:
obtaining a training scheme corresponding to the workload information as the training scheme of the first user.

18. An apparatus for processing personnel workload state based on multimodal data, comprising:
an acquisition module configured to acquire state information of a first user;
a determination module configured to determine workload information of the first user based on the acquired state information, wherein the state information comprises at least two of: physiological information, eye movement information, electroencephalogram information, brain function imaging information, motion capture information, spatiotemporal acquisition information, behavior acquisition information, and facial expression and state information; and
a processing module configured to feed back the workload information to a first management account, and formulate a training scheme matching the workload sate.

19. A system for processing personnel workload state based on multimodal data, wherein the system comprises the apparatus according to claim 18.

20. An electronic device, comprising:
a processor, and a memory, wherein one or more computer programs are stored in the memory, the one or more computer programs comprise instructions, and the electronic device, when executed by the processor, is configured to implement the method according to any one of claims 1 to 17.

21. A computer-readable storage medium on which a computer program is stored, wherein the computer program, when running on a computer, enable a computer to implement the method according to any one of claims 1 to 17.
